(19) Europäisches Patentamt
European Patent Office
Office européen des brevets

(11) Veröffentlichungsnummer: **0 101 598**
**B1**

# (12) EUROPÄISCHE PATENTSCHRIFT

(45) Veröffentlichungstag der Patentschrift: **20.04.88**

(51) Int. Cl.[4]: **C 07 D 205/08**, C 07 D 403/04, C 07 D 405/04, C 07 D 405/14

(21) Anmeldenummer: **83107910.8**

(22) Anmeldetag: **10.08.83**

(54) **Beta-Lactame.**

(30) Priorität: **19.08.82 CH 4961/82**
**01.07.83 CH 3643/83**

(43) Veröffentlichungstag der Anmeldung: **29.02.84 Patentblatt 84/9**

(45) Bekanntmachung des Hinweises auf die Patenterteilung: **20.04.88 Patentblatt 88/16**

(84) Benannte Vertragsstaaten: **AT BE CH DE FR GB IT LI LU NL SE**

(56) Entgegenhaltungen:
**EP - A - 0 073 061**

**JOURNAL OF ANTIBIOTICS, Band 35, Nr. 5, Mai 1982, Seiten 589-593, Tokyo, JP. NEIL S. ISAACS et al.: "The inhibition of bacterial beta-lactamases by some monocyclic beta-lactams"**
**CHEMICAL ABSTRACTS, Band 94, Nr. 17, 27. April 1981, Seite 736, Nr. 139522f, Columbus, Ohio, USA S.D. SHARMA et al.: "Some novel monocyclic cis-beta.lactams from glycine"**
**Chemical Abstracts 91, 91444r (1979)**

(73) Patentinhaber: **F. HOFFMANN-LA ROCHE & CO. Aktiengesellschaft, CH-4002 Basel (CH)**

(72) Erfinder: **Adam-Molina, Solange, Dr., 60 D, rue de Village-Neuf, F-68300 Rosenau (FR)**

(74) Vertreter: **Lederer, Franz, Dr. et al, Van der Werth, Lederer & Riederer Patentanwälte Lucile-Grahn-Strasse 22, D-8000 München 80 (DE)**

EP 0 101 598 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft optisch einheitliche β-Lactame der allgemeinen Formel

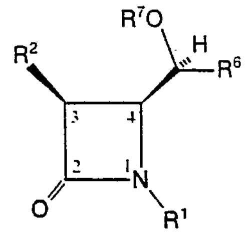

I

worin $R^1$ niederes 2- oder 1-Alkenyl oder eine Gruppe der Formel

$-CH_2-CH(OR^4)_2$ (a),

$-CH_2-CHO$ (b),

$-CO-CH(OH)_2$ (c) oder

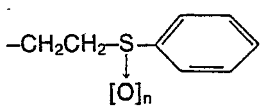

(d),

$R^4$ niederes Alkyl, n die Zahl 0 oder 1, $R^2$ Amino, Azido, Phthalimido, $ROCO-CH=C(CH_3)-NH-$ oder Z–NH–; R niederes Alkyl, Z eine leicht abspaltbare Acylgruppe und $R^6$ und $R^7$ je einen, gegebenenfalls miteinander verknüpften, gegebenenfalls Sauerstoff enthaltenden, über ein Kohlenstoffatom gebundenen niederen Kohlenwasserstoffrest bedeuten, wobei $R^1$ niederes 2- oder 1-Alkenyl oder eine der Gruppen (a), (c) und (d) bedeutet, wenn $R^2$ Amino bedeutet, und die mit nieder bezeichneten Reste bis zu 8 Kohlenstoffatome enthalten und die entsprechenden optischen Antipoden davon.

Die β-Lactame der allgemeinen Formel I sind dank ihrer besonderen, am $N_1$-Atom befindlichen Schutzgruppen wertvolle Zwischenprodukte zur besonders leichten Herstellung von β-Lactamen mit antimikrobiellen Eigenschaften, insbesondere von $N_1$-Sulfo-azetidinonen der weiter unten beschriebenen Formel IIa.

Zwischenprodukte der Formel I mit direkt abspaltbaren Schutzgruppen sind diejenigen der allgemeinen Formel

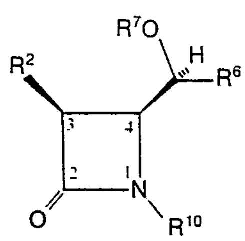

Ia

worin $R^2$ und $R^3$ die obige Bedeutung haben und $R^{10}$ niederes 1-Alkenyl oder die obige Gruppe (c) bedeutet.

Die übrigen Zwischenprodukte der Formel I, d.h. diejenigen worin $R^1$ eine andere Bedeutung als $R^{10}$ hat, sind Vorprodukte, welche – wie nachstehend eingehend ausgeführt werden wird – in die obigen Verbindungen der Formel Ia leicht übergeführt werden können.

Der Ausdruck «niederes Alkenyl» bedeutet eine olefinische Kohlenwasserstoffgruppe, die geradkettig oder verzweigt sein kann und bevorzugt bis zu 8, insbesondere bis zu 4 Kohlenstoffatome enthält, wie z.B. Vinyl, 2-Propenyl (Allyl), 1-Propenyl, Isopropenyl, 2-Methallyl, 1- oder 2-Butenyl, 1- oder 2-Hexenyl, 1- oder 2-Heptenyl, 1- oder 2-Octenyl usw. Der Ausdruck «niederes Alkyl» bedeutet, allein oder in Zusammensetzungen, wie «niederes Alkoxycarbonyl», eine gesättigte Kohlenwasserstoffgruppe, die geradkettig oder verzweigt sein kann und bevorzugt bis zu 8, insbesondere bis zu 4 Kohlenstoffatome enthält, wie z.B. Methyl, Aethyl, n-Propyl, Isopropyl, n-Butyl, Isobutyl, sec-Butyl, t-Butyl, n-Pentyl, Isopentyl, n-Hexyl, n-Heptyl, n-Octyl usw. «Niederes Alkoxy» hat analoge Bedeutung. «Niederes Cycloalkyl» bedeutet einen alicyclischen, gesättigten Kohlenwasserstoffrest mit 3–8 Kohlenstoffatomen, z.B. Cyclopropyl, Cyclobutyl, Cyclopentyl, Cyclohexyl, Cycloheptyl, Cyclooctyl. «Halogen» stellt alle 4 Halogene dar, insbesondere Chlor und Brom.

Die leicht abspaltbare Acylgruppe Z ist z.B. eine ggfs. halogensubstituierte niedere Alkoxycarbonylgruppe, wie t-Butoxycarbonyl oder Trichloräthoxycarbonyl; ferner auch Benzyloxycarbonyl und Benzhydryl.

$R^6$ kann beispielsweise niederes Alkyl, Phenyl-niederes Alkyl oder niederes Alkoxyalkyl, z.B. niederes Alkoxymethyl, und $R^7$ kann beispielsweise niederes Alkyl oder Phenyl-niederes Alkyl bedeuten. Diese Gruppen können zusammen mit dem Sauerstoffatom und dem Chiralitätszentrum auch einen 5- oder 6-gliedrigen O-Heterocyclus darstellen, der ggfs. ein weiteres mit dem Chiralitätszentrum nicht direkt verbundenes Sauerstoffatom enthält und ggfs. durch niederes Alkyl, niederes Alkoxy, Oxo oder Spirocyclo-niederes Alkyl substituiert sein kann. Beispiele sind:

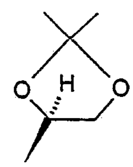

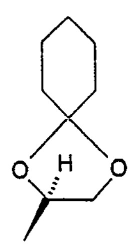

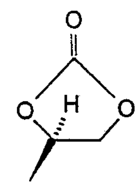

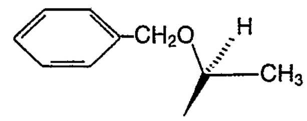

und die entsprechenden optischen Antipoden davon.

Bevorzugte Gruppen sind:

$R^1$: Vinyl, 2-Propenyl (Allyl), 1-Propenyl, 2,2-Dimethoxyäthyl, 2,2-Diäthoxyäthyl, Formylmethyl, Dihydroxyacetyl, 2-Phenylthioäthyl und 2-Phenylsulfinyläthyl, insbesondere 2- oder 1-Propenyl.

$R^2$: Amino, Phthalimido, Benzyloxycarbonylami-

no, t-Butoxycarbonylamino und 2-Methoxycarbonyl-1-methyl-vinylamino.

$R^6$, $R^7$: Zusammen mit dem Sauerstoffatom und dem Chiralitätszentrum die Gruppe

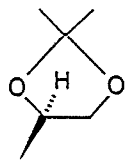

[(R)-2,2-Dimethyl-1,3-dioxolan-4-yl)].

Die Verbindungen der Formel I liegen in der optisch einheitlichen cis-Form vor.

Die β-Lactame der Formel I können erfindungsgemäss hergestellt werden, indem man ein reaktives Derivat einer Carbonsäure der allgemeinen Formel

$R^{20}$–$CH_2$–COOH III

worin $R^{20}$ Azido, Phthalimido oder die Gruppe ROCO–CH = C($CH_3$)–NH– und R niederes Alkyl bedeuten, in Gegenwart einer Base mit einer Verbindung der allgemeinen Formel

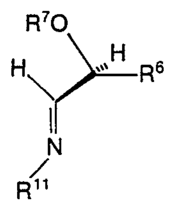

IV

worin $R^{11}$ niederes 2-Alkenyl oder eine der oben definierten Gruppen (a) und (d) bedeutet und $R^6$ und $R^7$ obige Bedeutung besitzen, oder dem entsprechenden optischen Antipoden davon zu einer Verbindung der allgemeinen Formel

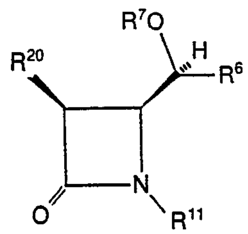

Ie

worin $R^{11}$, $R^{20}$, $R^6$ und $R^7$ obige Bedeutung besitzen, oder dem entsprechenden optischen Antipoden davon umsetzt und erwünschtenfalls eine als $R^{11}$ vorhandene niedere 2-Alkenylgruppe in die entsprechende 1-Alkenylgruppe oder eine als $R^{11}$ vorhandene Gruppe (a) in die Gruppe (b) oder (c) oder eine als $R^{11}$ vorhandene Gruppe (d) in die Vinylgruppe überführt, wobei man vorgängig oder anschliessend die Gruppe $R^{20}$ erwünschtenfalls in die Aminogruppe überführt und diese erwünschtenfalls mit einem eine leicht abspaltbare Acylgruppe abgebenden Mittel acyliert.

Bei der Reaktion eines reaktiven Derivates einer Carbonsäure der Formel III mit einer Verbindung der Formel IV bzw. dem optischen Antipoden davon handelt es sich um eine dem Fachmann geläufige Cycloaddition. Geeignete reaktive Carbonsäurederivate sind beispielsweise die entsprechenden Carbonsäurehalogenide, insbesondere die Carbonsäurechloride, entsprechende Carbonsäureanhydride und gemischte Anhydride (z.B. mit Trifluoressigsäure, Mesitylensulfonsäure und dergleichen), entsprechende Carbonsäureimidazolide und dergleichen. Zweckmässigerweise arbeitet man dabei in Gegenwart einer Base, beispielsweise eines tertiären Amins, wie Triäthylamin, und in einem inerten organischen Lösungsmittel, wobei insbesondere Äther, wie Tetrahydrofuran, Diäthyläther, t-Butylmethyläther, Dioxan, Aethylenglykoldimethyläther oder dergleichen, halogenierte Kohlenwasserstoffe, wie Methylenchlorid, Chloroform, 1,2-Dichloräthan oder dergleichen, Acetonitril, Dimethylformamid oder dergleichen, in Frage kommen. Die obige Cycloaddition wird dabei in einem Temperaturbereich von etwa −30 °C bis etwa 50 °C durchgeführt.

Bei der erwähnten Umsetzung erhält man eine Verbindung in der optisch einheitlichen cis-Form, d.h. eine Verbindung der allgemeinen Formel

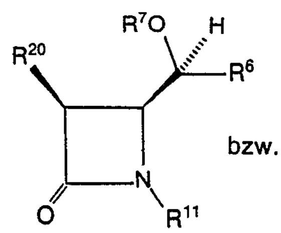

bzw.

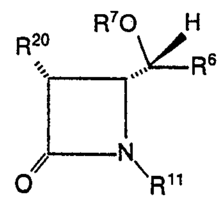

Ie[1] Ie[2]

worin $R^{11}$, $R^{20}$, $R^6$ und $R^7$ die obige Bedeutung haben.

Die als Ausgangsstoffe verwendeten Verbindungen der Formel IV bzw. deren optische Antipoden sind mit Ausnahme der in Chemical Abstracts 91, 91444r (1979) beschriebenen Verbindung ($R^{11}$ = Allyl, $R^6$ und $R^7$ zusammen mit dem Sauerstoffatom = –O–CH = CH–$CH_2$–$CH_2$–) neu und können hergestellt werden, indem man einen Aldehyd der allgemeinen Formel

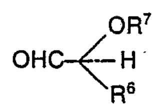

V

worin $R^6$ und $R^7$ die obige Bedeutung haben, mit einem Amin der allgemeinen Formel

$R^{11}$–$NH_2$ VI

worin $R^{11}$ die obige Bedeutung hat, umsetzt. Man arbeitet dabei vorzugsweise in einem inerten organischen Lösungsmittel, beispielsweise in einem halogenierten Kohlenwasserstoff, wie Methylenchlorid, Chloroform, 1,2 Dichloräthan und dergleichen, oder in einem Kohlenwasserstoff, wie Benzol, Toluol und dergleichen. Vorzugsweise entfernt man dabei laufend das während der Reaktion entstehende Reaktionswasser, beispielsweise durch azeotrope Destillation oder durch Arbeiten in Gegenwart eines wasserentziehenden Mittels, beispielsweise in Gegenwart eines geeigneten Molekularsiebes, oder in Gegenwart von anderen herkömmlichen Trocknungsmitteln, wie Kaliumcarbonat, Magnesiumsulfat und dergleichen. Bei azeotroper Entfernung des gebildeten Reaktionswassers arbeitet man bei der Siedetemperatur des gewählten Lösungsmittels; bei Verwendung einers wasserentziehenden Mittels arbeitet man vorzugsweise bei Raumtemperatur.

Die in der erhaltenen Verbindung der Formel Ie vorhandenen N-Schutzgruppen $R^{11}$ sind nicht an sich abspaltbar, können aber wie folgt in abspaltbare Schutzgruppen $R^{10}$ (vgl. obige Formel Ia) übergeführt werden:

I. $R^{11}$: niederes 2-Alkenyl → $R^{10}$; niederes 1-Alkenyl, z.B. $-CH_2-CH=CH_2 \rightarrow -CH=CH-CH_3$.

Diese Isomerisierung erfolgt vorteilhaft mit Hilfe eines Isomerisierungskatalysators, z.B. mit einem Palladiumdihalogenid, wie Palladiumdichlorid oder mit einem Tris-(triphenylphosphin)-rhodium(I)-halogenid, z.B. mit dem entsprechenden Chlorid, oder auch mit Palladiumkohle und einer Protonensäure, wie Salzsäure oder Phosphorsäure. Die Isomerisierung wird vorteilhaft in einem inerten organischen Lösungsmittel, wie Aethanol, Methylenchlorid bzw. Mischungen davon mit Wasser, und bei einer Temperatur zwischen etwa 50 °C und dem Siedepunkt des Reaktionsgemisches durchgeführt.

Die Umsetzung (b) → (e) erfolgt durch Umsetzung mit einem sekundären niederen, aliphatischen oder cycloaliphatischen Amin, z.B. mit Morpholin, Pyrrolidin, Piperidin, 4-Methylpiperidin, Diäthylamin, insbesondere mit Morpholin. Diese Umsetzung erfolt vorzugsweise in einem inerten organischen Lösungsmittel, wie Methylenchlorid oder Chloroform, und bei einer Temperatur zwischen etwa 0 °C und dem Siedepunkt des Reaktionsgemisches; bevorzugt ist die Raumtemperatur.

II. $R^{11}$: Gruppe (a) → $R^1$: Gruppe (b) → $R_{10}$: Gruppe (c) $-CH_2-CH(OR^4)_2 \rightarrow -CH_2-CHO \rightarrow -CO-CH(OH)_2$.

Die Umwandlung (a) → (b) erfolgt vorzugsweise mit einem Tri-niederalkyljodsilan, insbesondere Trimethyljodsilan, oder mit p-Toluolsulfonsäure. Die Umsetzung erfolgt vorzugsweise in einem inerten organischen Lösungsmittel, wie Acetonitril, und bei einer Temperatur zwischen etwa 0 °C und 50 °C.

Die Oxidation (b) → (c) erfolgt vorzugsweise mit Selendioxid unter sauren Bedingungen, z.B. in Gegenwart von Essigsäure. Die Umsetzung erfolgt vorzugsweise in einem inerten organischen Lösungsmittel, wie Dioxan, und bei einer Temperatur zwischen der Raumtemperatur und dem Siedepunkt des Reaktionsgemisches.

III. $R^{11}$: Gruppe (d) → $R^{10}$: Vinyl

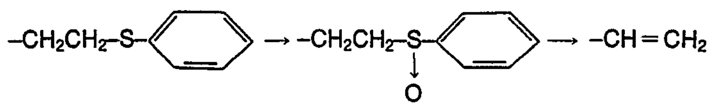

Die erste Stufe, die Oxidation der Phenylthioäthylgruppe zu Phenylsulfinyläthyl, erfolgt durch Behandlung mit einem organischen oder anorganischen Oxidationsmittel. Als Oxidationsmittel dienen verschiedene, Sauerstoff leicht abgebende Verbindungen, wie z.B. organische Peroxide, z.B. monosubstituierte organische Peroxide, wie $C_1-C_4$ Alkyl- oder Alkanoylhydroperoxide, wie t-Butylhydroperoxid, Perameisensäure, Peressigsäure; sowier phenylsubstituierte Derivate dieser Hydroperoxide, wie Cumolhydroperoxid, Perbenzoesäure. Der Phenylsubstituent kann gegebenenfalls eine weitere niedere Gruppe, z.B. $C_1-C_4$ Alkyl oder Alkoxy, Halogen oder Carboxygruppen tragen, z.B. 4-Methylperbenzoesäure, 4-Methoxyperbenzoesäure, 3-Chlorperbenzoesäure, Monoperphthalsäure. Als Oxidationsmittel können auch verschiedene anorganische Oxidationsmittel verwendet werden, z.B. Wasserstoffperoxid; Ozon; Permanganate, wie Kalium- oder Natriumpermanganat; Hypochlorite, wie Natrium-, Kalium- oder Ammoniumhypochlorit; Peroxymono- und Peroxydischwefelsäure. Bevorzugt ist die Verwendung von 3-Chlorperbenzoesäure. Die Oxidation erfolgt mit Vorteil in einem inerten Lösungsmittel, z.B. in einem aprotischen inerten Lösungsmittel, wie Tetrahydrofuran, Dioxan, Methylenchlorid, Chloroform, Aethylacetat oder Aceton; oder in einem protischen Lösungsmittel, wie Wasser, einem niederen Alkanol, z.B. Methanol, Aethanol, oder einer niederen, ggf. halogenierten Alkancarbonsäure, z.B. Ameisensäure, Essigsäure, Trifluoressigsäure. Die Reaktionstemperatur bewegt sich vornehmlich im Bereiche von etwa −20 °C bis +75 °C.

Der Abbau der Phenylsulfinyläthylgruppe zur Vinylgruppe erfolgt durch Erhitzen auf etwa 100–200 °C, vorzugsweise in einem aprotischen organischen Lösungsmittel wie Hexamethylenphosphorsäuretriamid, Dimethylsulfoxid oder Dimethylformamid, Benzol oder Toluol. Die Reaktion kann durch Zusatz eines abfängers für die bei der Reaktion gebildeten Phenylsulfonsäure beschleunigt werden, wobei geeignete Agenzien insbesondere Trimethylphosphit oder Propiolsäureester, wie z.B. Propiolsäuremethylester, darstellen.

Die Umwandlung der Gruppe $R^{20}$ in die Aminogruppe erfolgt nach an sich bekannten und jedem Fachmann geläufigen Methoden, wobei die anzuwendende Methode von der Natur der Gruppe $R^{20}$ abhängt. Die Phthalimidogruppe kann beispielsweise durch Umsetzen mit Hydrazin, Methylhydrazin oder dergleichen entfernt werden, wobei man zweckmässigerweise in einem inerten organischen Lösungsmittel arbeitet. Geeignete Lösungsmittel sind z.B. halogenierte Kohlenwasserstoffe, wie Methylenchlorid, Chloroform und dergleichen, Äther, wie Tetrahydrofuran, Dioxan, t-Butylmethyläther und dergleichen, usw. Die Azidogruppe kann beispielsweise mit elementarem Wasserstoff in Gegenwart eines Katalysators, wie Palladiumkohle, Raney-Nickel, Platinoxid und dergleichen, oder auch durch Einwirken von Schwefelwasserstoff und einem tertiären Amin, wie Triäthylamin, zur Aminogruppe reduziert werden. Die Gruppe ROCO–CH = C($CH_3$)–NH– kann beispielsweise durch milde saure Hydrolyse in die Aminogruppe übergeführt werden.

Die Acylierung der Aminogruppe mit einem eine leicht abspaltbare Acylgruppe abgebenden Mittel, beispielsweise Di-t-butyldicarbonat oder Chlorameisensäureester, wie Chlorameisensäurebenzylester, Chlorameisensäure-t-butylester, Chlorameisensäure-2,2,2-trichloräthylester und dergleichen, erfolgt zweckmässigerweise in einem inerten organischen Lösungsmittel, beispielsweise in einem halogenierten Kohlenwasserstoff, wie Methylenchlorid, Chloroform und dergleichen, und zweckmässigerweise in Gegenwart eines säurebindenden Mittels, wie Butylenoxid, Triäthylamin, Chinuclidin, usw. Zweckmässigerweise arbeitet man bei Raumtemperatur.

Zur Herstellung der antimikrobiell wirksamen β-Lactame der Formel IIa wird zunächst in einer erhaltenen Verbindung der allgemeinen Formel

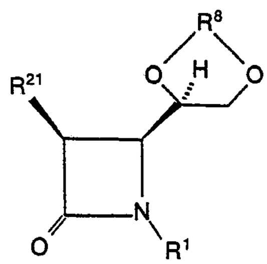

Ik

in der $R^{21}$ Azido, Phthalimido, ROCO–CH = C($CH_3$)–NH– oder Z–NH– und $R^8$ Methylen, Aethylen, Oxomethylen, Cyclohexyliden oder, vorzugsweise, Isopropyliden bedeuten, und $R^1$, R und Z die obige Bedeutung haben, bzw. dem optischen Antipoden davon die Gruppe $R^8$ abspaltet, z.B. durch Behandeln mit einem mild sauren Agens, wie z.B. mit einem sulfonierten Ionenaustauscher, Pyridinium-p-toluolsulfonat oder p-Toluolsulfonsäure, in einem niederen Alkanol, wie Methanol oder Aethanol, oder in wässrigem Tetrahydrofuran, vorzugsweise bei Raumtemperatur bis etw 80 °C. Man erhält ein optisch aktives Diol der allgemeinen Formel

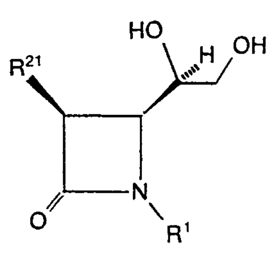

Im

in der $R^1$ und $R^{21}$ die obige Bedeutung haben, bzw. den optischen Antipoden davon.

In der so erhaltenen Verbindung der Formel Im bzw. dem optischen Antipoden davon wird die Diolgruppierung gespalten, wobei ein Aldehyd der allgemeinen Formel

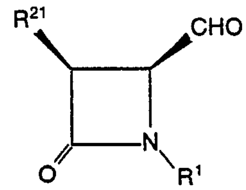

In

in der $R^1$ und $R^{21}$ die obige Bedeutung haben, bzw. der optische Antipode davon erhalten wird. Diese Spaltung erfolt nach an sich bekannten Methoden und kann z.B. mit Alkalimetallperjodat, wie Natriummetaperjodat, in Wasser, ggfs. im Gemisch mit z.B. Tetrahydrofuran oder einem niederen Alkanol, wie Methanol, bewerkstelligt werden. Die Aldehydgruppen des Aldehyds In bzw. des optischen Antipoden davon kann man nach bekannten Methoden reduzieren, z.B. durch Behandeln mit einem komplexen Metallhydrid, wie Natriumborhydrid, in einem niederen Alkanol, wie Aethanol, Isopropanol oder dergleichen. Man erhält einen Alkohol der allgemeinen Formel

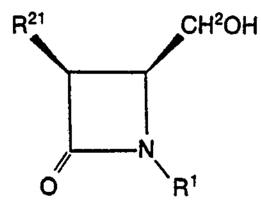

Io

in der $R^1$ und $R^{21}$ die obigen Bedeutungen besitzen, bzw. den optischen Antipoden davon.

Durch Umwandlung der 4-Formyl- bzw. 4-Hydroxymethylgruppe in die 4-Carbamoyl- bzw. 4-Carbamoyloxymethylgruppe in dem Fachmann bekannter Weise (vgl. die nachstehenden Schemata I–III) erhält man eine Verbindung der nachstehenden Formel Ir.

In der erhaltenen Verbindung der allgmeinen Formel

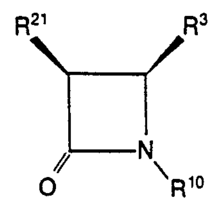

Ir

in der $R^{10}$. $R^{21}$ und $R^3$ die obige Bedeutung haben, wird die N-Schutzgruppe $R^{10}$ abgespalten. Niedere 1-Alkenylgruppen, z.B. 1-Propenyl oder Vinyl, werden oxidativ abgespalten, insbesondere durch Behandeln mit einem Alkalimetallpermanganat, z.B. Kaliumpermanganat, vorzugsweise einer wässrigen Lösung von Kaliumpermanganat. Wahlweise kann man die Oxidation mit Hilfe eines Alkalimetallperjodats, z.B. Kaliumperjodat, in Gegenwart einer katalytischen Menge des erwähnten Alkalimetallpermanganats durchführen. Die Reaktion erfolgt bevorzugt in einem wässrigen, gepufferten, insbesondere auf pH 7–8 gepufferten

Medium, kann aber auch in einem mit Wasser mischbaren organischen Lösungsmittel, z.B. in Aceton, Dimethoxyäthan, Dioxan oder Tetrahydrofuran, unter Zusatz einer schwachen organischen Base, wie Pyridin, oder in einer Mischung eines dieser Lösungsmittel mit dem erwähnten wässrigen Puffer, durchgeführt werden. Wahlweise kann die Reaktion ebenfalls unter Phasentransferkatalyse durchgeführt werden, d.h. in Gegenwart einer wässrigen bzw. nichtwässrigen Phase, z.B. der obige wässrige Puffer sowie ein mit Wasser nicht mischbares, inertes organisches Lösungsmittel, wie z.B. Methylenchlorid oder Benzol. Als Phasentransferkatalysatoren können die üblicherweise hierfür verwendeten Agenzien eingesetzt werden, insbesondere organische quaternäre Ammoniumhalogenide, wie Benzyltriäthylammoniumchlorid, Tetra-n-butylammoniumbromid und Cetyltrimethylammoniumbromid. Die oxidative Abspaltung von 1-Alkenylgruppen wird vorzugsweise bei einer Temperatur im Bereiche zwischen etwa 0 °C und 25 °C durchgeführt.

Die Hydroxyacetylgruppe [$R^{10}$ als Gruppe (c)] wird bevorzugt mit einer wässrigen starken Base, z.B. mit wässrigem Ammoniak oder wässriger Alkalilauge, in einem inerten organischen Lösungsmittel, z.B. einem halogenierten Kohlenwasserstoff, wie Chloroform, Kohlenstofftetrachlorid oder Methylenchlorid, bei einer Temperatur zwischen etwa 0 °C und +50 °C, abgespalten.

Nach Entfernung der obigen Schutzgruppe $R^{10}$ kann durch Umsetzung mit dem reaktionsfähigen Derivat von Schwefeltrioxid die Gruppe $-SO_3H$ in Stellung 1 eingeführt werden, z.B. durch Umsetzung mit Komplexen von Schwefeltrioxid und einer Base, wie Pyridin, Trimethylamin, Picolin, Dimethylformamid usw., bei etwa 0–80 °C, in einem inerten organischen Lösungsmittel, z.B. in einem Äther, wie Dioxan, in Pyridin, Dimethylformamid usw. In Stellung 3 kann, vorgängig oder nach letzterer Umsetzung, die Aminogruppe $R^2$ durch Umwandlung der Gruppe $R^{20}$ (in obiger Weise) bzw. durch Abspaltung der Schutzgruppe Z freigesetzt werden; z.B. können Aralkoxycarbonylgruppen (insbesondere Benzyloxycarbonyl) oder die Benzhydrylgruppe hydrogenolytisch, z.B. durch Einwirken von Wasserstoff und Palladiumkohle, abgespalten werden; die t-Butoxycarbonylgruppe kann durch Behandeln mit Trifluoressigsäure oder Ameisensäure, die Trichloräthoxycarbonylgruppe wiederum durch Behandeln mit Zink und einer Protonensäure, wie Essigsäure oder Chlorwasserstoffsäure, abgespalten werden. Anschliessend kann die freigesetzte Aminogruppe $R^2$ mit einer entsprechend substituierten Carbonsäure bzw. einem reaktionsfähigen funktionellen Derivat davon (z.B. Säureanhydrid, Säureamid, aktivem Ester, z.B. einem Thioester, wie dem Benzthiazolylthioester) acyliert werden, wobei die verschiedensten Acylgruppen, wie sie z.B. aus der Penicillin- oder Cephalosporinchemie bekannt sind, eingeführt werden können. Beispielsweise können in dieser Weise optisch einheitliche, antimikrobiell wirksame β-Lactame der allgemeinen Formel

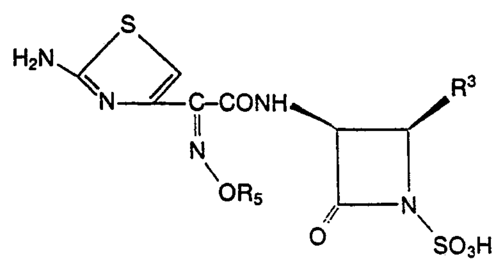

IIa

worin $R^5$ Wasserstoff, niederes Alkyl oder Carboxy-niederes Alkyl und $R^3$ Carbamoyl oder Carbamoyloxymethyl darstellt, bzw. die entsprechenden optischen Antipoden davon nach an sich bekannten Methoden hergestellt werden.

Beispiele für entsprechende Überführungen in Endprodukte sind in den nachstehenden Reaktionsschemata I und II aufgeführt.

Schema I

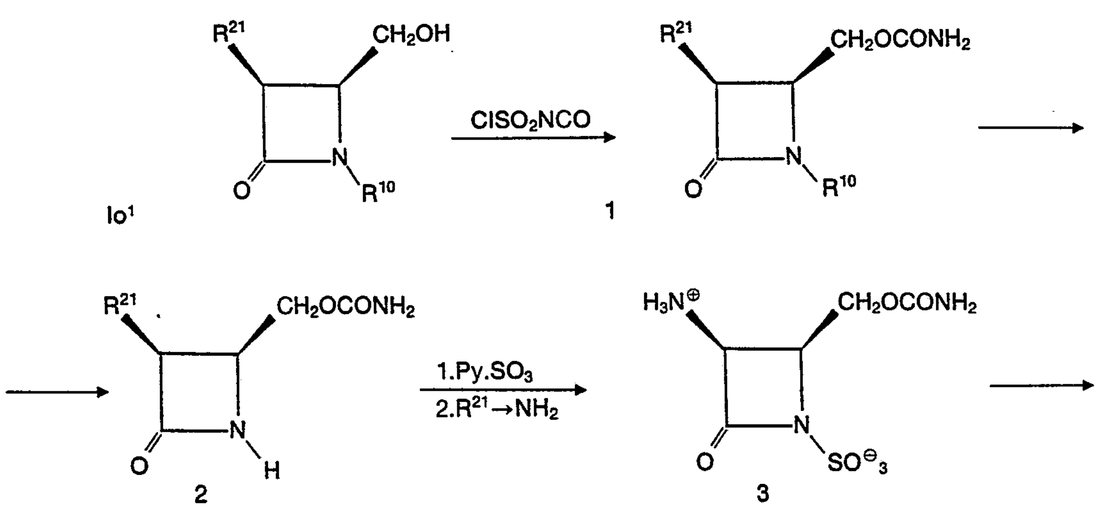


Io¹ 1 2 3

**Schema I** (Fortsetzung)

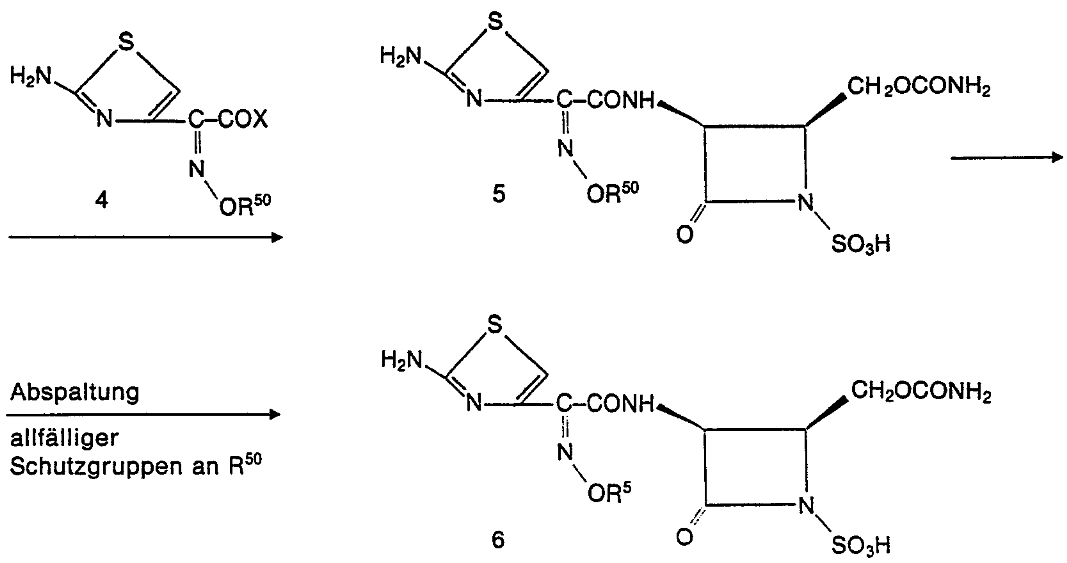


4

5

6

Schema II

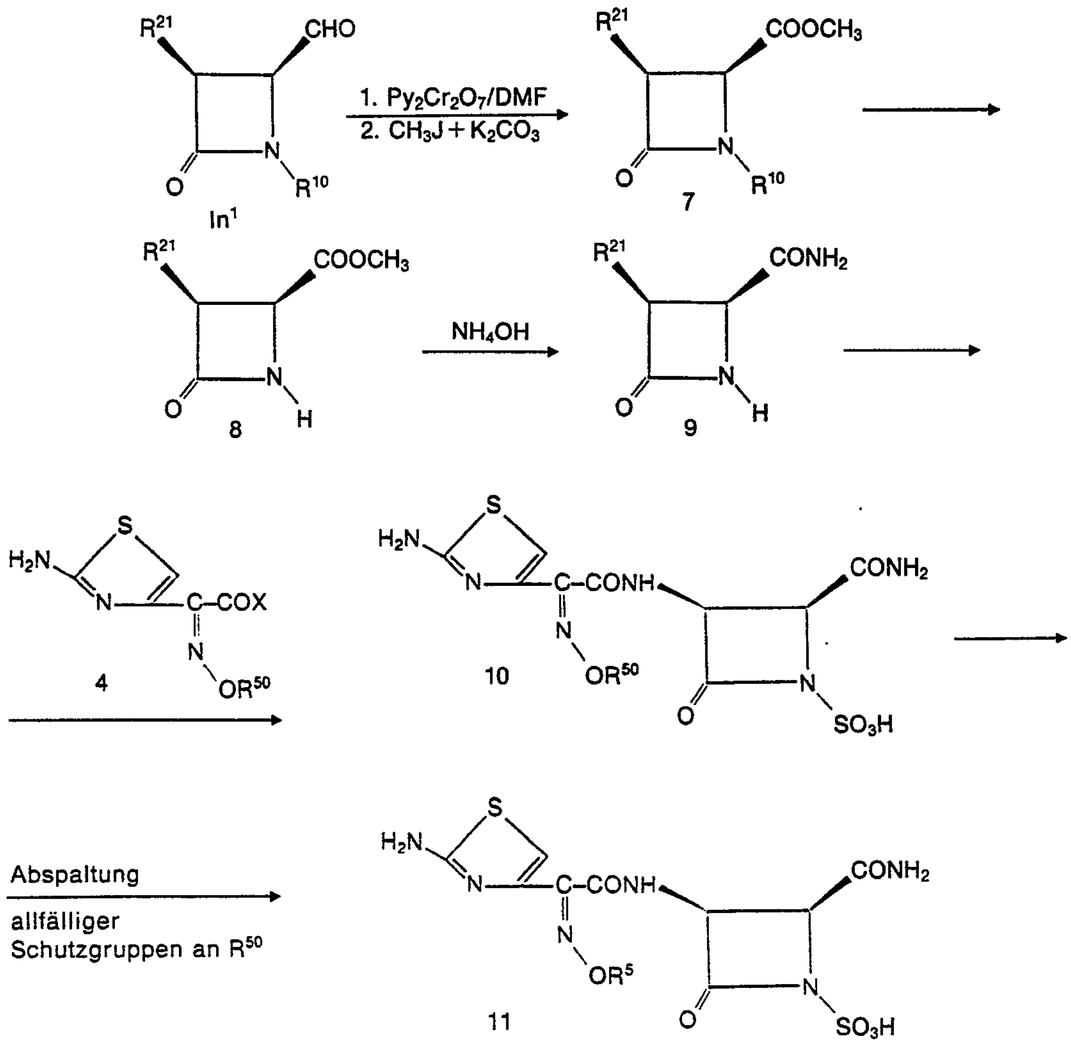


In[1]

7

8

9

4

10

11

Erklärungen zu den Schemata I, II und III

DMF = Dimethylformamid

Py = Pyridin

Py · $SO_3$ = Schwefeltrioxid-pyridiniumkomplex

COX = reaktionsfähiges Derivat einer Carbonsäure, z.B. Säureanhydrid, Säureamid, aktiver Ester, z.B. Benzthiazolylester

$R^{50}$ = Wasserstoff, niederes Alkyl (z.B. Methyl), geschütztes Carboxy-niederes Alkyl, z.B. geschütztes Carboxymethyl, geschütztes 1-Methyl-1-carboxy-äthyl. Schutzgruppe: z.B. t-Butyl (mit z.B. Trifluoressigsäure abspaltbar), Benzyl und p-Nitrobenzyl (mit z.B. Wasserstoff und Palladiumkohle abspaltbar), 2-Trimethylsilyl)-äthyl (mit z.B. Tetrabutylammoniumfluorid abspaltbar)

$R^5$ = Wasserstoff, niederes Alkyl, (z.B. Methyl), Carboxy-niederes Alkyl, z.B. Carboxymethyl, 1-Methyl-1-carboxy-äthyl

$R^{10}$, $R^{21}$ = obige Bedeutung.

In den Schemata I und II nicht näher erläuterte Reaktionen werden in der oben im Detail beschriebenen Weise ausgeführt.

Die nachfolgenden Beispiele erläutern die vorliegende Erfindung. Alle Temperaturen sind in Celsiusgraden angegeben. Ar = Aromat.

Beispiel 1

Zu einer bei Raumtemperatur gerührten Lösung von 18,6 ml (0,25 Mol) Allylamin in 500 ml Methylenchlorid gibt man 50 g Molekularsieb 4 Å und nach 20 Minuten 32,5 g (0,25 Mol) Isopropyliden-L-glyceraldehyd und 100 g Magnesiumsulfat. Anschliessend rührt man noch während 1 Stunde bei Raumtemperatur. Die organische Lösung von Isopropyliden-L-glyceraldehyd-allylimin wird unter Argon auf −30° gekühlt und unter Rühren mit 31,3 ml (0,25 Mol) Triäthylamin versetzt. Dann wird innerhalb 2 Stunden eine Lösung von 45,25 g (0,25 Mol) Phthaloylglycylchlorid in 200 ml trockenem Methylenchlorid zugetropft und anschliessend 12 Stunden bei Raumtemperatur weitergerührt. Das Reaktionsgemisch wird dreimal mit je 700 ml Wasser und 700 ml Kochsalzlösung gewaschen und über Magnesiumsulfat getrocknet, filtriert und eingedampft. Der Rückstand wird mit Cyclohexan-Aethylacetat (6:4) an Kieselgel chromatographiert. Man erhält 50 g N-[cis-(3S,4S)-1-Allyl-4-[(R)-2,2-dimethyl-1,3-dioxolan-4-yl)-2-oxo-3-azetidinyl]-phthalimid als Öl. $[\alpha]^{20}_D$ = +115° (0,13 in Chloroform).

NMR ($CDCl_3$) δ (ppm): 1,28 (3H, s), 1,41 (3H, s), 3,36–4,5 (6H), 5,87 (1H, d, 5 Hz), 5,18–5,5 (2H, m, $CH_2$=), 5,68–6,16 (1H, m, CH=), 7,87 (4H, m, Ar)

Beispiel 2

Eine Suspension von 25 g (70,2 mMol) N-[cis-(3S,4S)-1-Allyl-4-[(R)-2,2-dimethyl-1,3-dioxolan-4-yl)-2-oxo-3-azetidinyl]-phthalimid und 0,5 g Palladiumdichlorid in 300 ml Methylenchlorid und 30 ml Wasser wird unter Rühren 3 Stunden unter Rückflussbedingungen erhitzt. Nach dem Erkalten und Filtrieren wird die organische Phase abgetrennt, mit Wasser gewaschen, über Magnesiumsulfat getrocknet, filtriert und eingedampft. Man erhält 24 g N-[cis-(3S,4S)-4-[(R)-2,2-dimethyl-1,3-dioxolan-4-yl)-2-oxo-1-(1-propenyl)-3-azetidinyl]-phthalimid als Schaum. $[\alpha]^{20}_D$ = +77,7° (c = 0,153 in Chloroform).

NMR ($CDCl_3$) δ (ppm): 1,26 (3H, s, $CH_3$), 1,42 (3H, s, $CH_3$), 1,71 (3H, dd, 1,5 und 6,5 Hz, $CH_3$), 3,34–3,76 (2H, m, $CH_2$), 3,97–4,52 (2H, m, $H_4$ und CH), 5,38 (1H, d, 5,5 Hz, $H_3$), 5,67–6,05 (1H, m =CH–$CH_3$), 6,4 (1H, dd, 1,5 und 14 Hz, =CH–N–), 7,82 (4H, m, Ar)

Beispiel 3

Eine Lösung von 0,356 g (1 mMol) N-[cis-(3S,4S)-4-[(R)-2,2-dimethyl-1,3-dioxolan-4-yl]-2-oxo-1-(1-propenyl)-3-azetidinyl]-phthalimid und 0,26 ml Pyridin in 50 ml Aceton und 50 ml Wasser wird unter Rühren bei 5° mit einer Lösung von 0,22 g (1,4 mMol) Kaliumpermanganat in 50 ml Aceton und 50 ml Wasser tropfenweise versetzt. Nach 3 Stunden filtriert man und dampft das Filtrat ein. Der Rückstand wird mit Cyclohexan-Aethylacetat (6:4) an Kieselgel chromatographiert. Man erhält 0,29 g N-[cis-(3S,4S)-4-[(R)-2,2-dimethyl-1,3-dioxolan-4yl]-2-oxo-3-azetidinyl]-phthalimid als Schaum.

NMR ($CDCl_3$) δ (ppm): 1,3 (3H, s, $CH_3$), 1,45 (3H, s, $CH_3$), 3,3–4,5 (4H, m), 5,4 (1H, d, 5,5 Hz, $H_3$), 7,0 (1H, s, –NH), 7,9 (4H ar)

Beispiel 4

Eine Lösung von 20 g (56 mMol) N-[cis-(3S,4S)-4-[(R)-2,2-dimethyl-1,3-dioxolan-4-yl]-2-oxo-1-(1-propenyl)-3-azetidinyl]-phthalimid in 200 ml Methylenchlorid wird mit 5,9 ml (119 mMol) Methylhydrazin versetzt. Das Reaktionsgemisch wird unter 2-stündigem Rühren auf 60° erhitzt. Nach dem Erkalten und Filtrieren wird das Filtrat eingedampft. Der Rückstand wird in 200 ml Methylenchlorid gelöst und mit 14,6 ml Butylenoxid versetzt. Unter Rühren bei 20° versetzt man das Reaktionsgemisch tropfenweise mit 12 ml Carbobenzoxychlorid und rührt über Nacht. Das Reaktionsgemisch wird dreimal mit je 200 ml Wasser gewaschen und über Magnesiumsulfat getrocknet, filtriert und eingedampft. Der Rückstand wird mit 200 ml Äther versetzt und der kristalline Niederschlag abgenutscht. Man erhält 14,5 g Benzyl-cis-(3S,4S)-4-[(R)-2,2-dimethyl-1,3-dioxolan-4-yl)-2-oxo-1-(1-propenyl)-3-azetidincarbamat. $[\alpha]^{20}_D$ = +8 (c = 1 in Chloroform).

NMR ($CDCl_3$) δ (ppm): 1,35 (3H, s, $CH_3$), 1,43 (3H, s, $CH_3$), 1,6 (3H, d, 6 Hz, $CH_3$), 3,68–4,32 (4H, m), 5,03–5,2 (3H, m), 5,5–6,3 (3H, m), 7,3 (5H, Ar).

Beispiel 5

Eine Lösung von 32,7 g (90,8 mMol) Benzyl-cis-(3S,4S)-4-[(R)-2,2-dimethyl-1,3-dioxolan-4-yl)-2-oxo-1-(1-propenyl)-3-azetidincarbamat und 25 ml Pyridin in 150 ml Aceton, 75 ml Wasser und 150 ml Dimethoxyäthan wird unter Rühren bei 20° innerhalb 90 Minuten mit 27,5 g (173,7 mMol) Kaliumpermanganat versetzt. Die Innentemperatur des

Reaktionsgefässes wird unterhalb 50° gehalten. Man filtriert über Kieselgur und dampft das Filtrat ein. Der Rückstand wird in Methylenchlorid gelöst, über Magnesiumsulfat getrocknet, filtriert und das Filtrat eingedampft. Der kristalline Rückstand wird in Äther aufgenommen und abgenutscht. Man erhält 20 g Benzyl-cis-(3S,4S)-4-[(R)-2,2-dimethyl-1,3-dioxolan4-yl]-2-oxo-3-azetidin-carbamat.

**Patentansprüche für die Vertragsstaaten: BE, CH, DE, FR, GB, IT, LI, LU, NL, SE.**

1. Optisch einheitliche β-Lactame der allgemeinen Formel

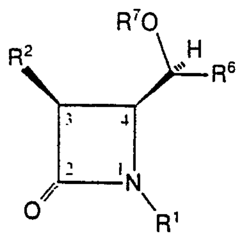

I

worin $R^1$ niederes 2- oder 1-Alkenyl oder eine Gruppe der Formel

$-CH_2-CH(OR^4)_2$ (a),

$-CH_2-CHO$ (b),

$-CO-CH(OH)_2$ (c) oder

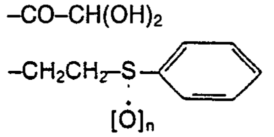

(d),

$R^4$ niederes Alkyl, n die Zahl 0 oder 1, $R^2$ Amino, Azido, Phthalimido, ROCO–CH = $C(CH_3)$–NH– oder Z-NH–; R niederes Alkyl, Z eine leicht abspaltbare Acylgruppe und $R^6$ und $R^7$ je einen, gegebenenfalls miteinander verknüpften, gegebenenfalls Sauerstoff enthaltenden, über ein Kohlenstoffatom gebundenen niederen Kohlenwasserstoffrest bedeuten, wobei $R^1$ niederes 2- oder 1-Alkenyl oder eine der Gruppen (a), (c) und (d) bedeutet, wenn $R^2$ Amino bedeutet, und die mit nieder bezeichneten Reste bis zu 8 Kohlenstoffatome enthalten und die entsprechenden optischen Antipoden davon.

2. Verbindungen gemäss Anspruch 1, dadurch gekennzeichnet, dass $R^1$ Vinyl, 2-Propenyl, 1-Propenyl, 2,2-Dimethoxyäthyl, 2,2-Diäthoxyäthyl, Formylmethyl, Dihydroxyacetyl, 2-Phenylthioäthyl oder 2-Phenylsulfinyläthyl bedeutet.

3. Verbindungen gemäss Anspruch 2, dadurch gekennzeichnet, dass $R^1$ 2- oder 1-Propenyl bedeutet.

4. Verbindungen gemäss einem der Ansprüche 1 bis 3, dadurch gekennzeichnet, dass $R^2$ Amino, Phthalimido, Benzyloxycarbonylamino, t-Butoxycarbonylamino oder 2-Methoxycarbonyl-1-methylvinylamino bedeutet.

5. Verbindungen gemäss einem der Ansprüche 1 bis 4, dadurch gekennzeichnet, dass $R^6$ niederes Alkyl, niederes Phenylalkyl oder niederes Alkoxyalkyl und $R^7$ niederes Alkyl oder niederes Phenylalkyl oder $R^6$ und $R^7$ zusammen mit dem Sauerstoffatom und dem Chiralitätszentrum einen 5- oder 6-gliedrigen, gegebenenfalls ein weiteres, mit dem Chiralitätszentrum nicht direkt verbundenes Sauerstoffatom enthaltenden und gegebenenfalls durch niederes Alkyl, niederes Alkoxy, Oxo oder niederes Spirocycloalkyl substituierten O-Heterocyclus bedeuten.

6. Verbindungen gemäss Anspruch 5, dadurch gekennzeichnet, dass $R^6$ und $R^7$ zusammen mit dem Sauerstoffatom und dem Chiralitätszentrum die Gruppe

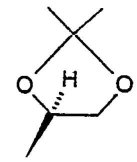

[(R)-2,2-Dimethyl-1-3,-dioxolan-4-yl] bedeuten.

7. N-[cis-(3S,4S)-1-Allyl-4-[(R)-2,2-dimethyl-1,3-dioxolan-4-yl)-2-oxo-3-azetidinyl]phthalimid.

8. N-[cis-(3S,4S)-4-[(R)-2,2-dimethyl-1,3-dioxolan-4-yl)-2-oxo-1-(1-propenyl)-3-azetidinyl]phthalimid.

9. Benzyl-cis-(3S,4S)-4-[(R)-2,2dimethyl-1,3-dioxolan-4-yl)-2-oxo-1-(1-propenyl)-3-azetidincarbamat.

10. Verfahren zur Herstellung von Verbindungen gemäss einem der Ansprüche 1 bis 9, dadurch gekennzeichnet, dass man ein reaktives Derivat einer Carbonsäure der allgemeinen Formel

$$R^{20}-CH_2-COOH \qquad III$$

worin $R^{20}$ Azido, Phthalimido oder die Gruppe ROCO–CH = $C(CH_3)$–NH– und R niederes Alkyl bedeuten, in Gegenwart einer Base mit einer Verbindung der allgemeinen Formel

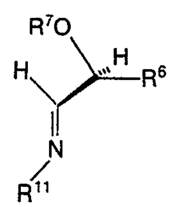

IV

worin $R^{11}$ niederes 2-Alkenyl oder eine der in Anspruch 1 definierten Gruppen (a) und (d) bedeutet und $R^6$ und $R^7$ die in Anspruch 1 angegebene Bedeutung besitzen, oder dem entsprechenden optischen Antipoden davon zu einer Verbindung der allgemeinen Formel

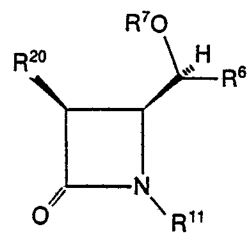

Ie

worin $R^{11}$ und $R^{20}$ obige und $R^6$ und $R^7$ die in Anspruch 1 angegebene Bedeutung besitzen,

oder dem entsprechenden otpischen Antipoden davon umsetzt und erwünschtenfalls eine als $R^{11}$ vorhandene niedere 2-Alkenylgruppe in die entsprechende 1-Alkenylgruppe oder eine als $R^{11}$ vorhandene Gruppe (a) in die Gruppe (b) oder (c) oder eine als $R^{11}$ vorhandene Gruppe (d) in die Vinylgruppe überführt, wobei man vorgängig oder anschliessend die Gruppe $R^{20}$ erwünschtenfalls in die Aminogruppe überführt und diese erwünschtenfalls mit einem eine leicht abspaltbare Acylgruppe abgebenden Mittel acyliert.

11. Verwendung von Verbindungen gemäss einem der Ansprüche 1 bis 9 zur Herstellung von antimikrobiell wirksamen, optisch einheitlichen β-Lactamen der allgemeinen Formel

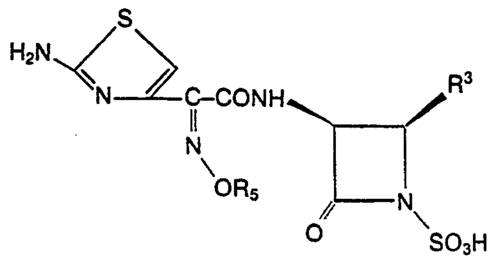

IIa

worin $R^3$ Carbamoyl oder Carbamoyloxymethyl und $R^5$ Wasserstoff, niederes Alkyl oder Carboxy-niederes Alkyl bedeuten, und den entsprechenden optischen Antipoden davon.

12. Verwendung von Verbindungen gemäss einem der Ansprüche 1 bis 9 zur Herstellung von antimikrobiell wirksamen, optisch einheitlichen β-Lactamen gemäss Anspruch 11, worin $R^3$ Carbamoyloxymethyl und $R^5$ Carboxymethyl bedeuten.

13. Verbindungen der allgemeinen Formel

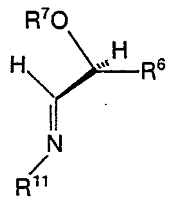

IV

worin $R^{11}$ niederes 2-Alkenyl oder eine der in Anspruch 1 definierten Gruppen (a) und (d) bedeutet und $R^6$ und $R^7$ die in Anspruch 1 angegebene Bedeutung besitzen, wobei $R^6$ und $R^7$ zusammen mit dem Sauerstoffatom nicht $-O-CH=CH-CH_2-CH_2-$ bedeuten, wenn $R^{11}$ Allyl bedeutet, und die entsprechenden optischen Antipoden davon.

14. Verbindungen gemäss Anspruch 13, dadurch gekennzeichnet, dass $R^{11}$ 2-Propenyl, 2,2-Dimethoxyäthyl, 2,2-Diäthoxyäthyl, 2-Phenylthioäthyl oder 2-Phenylsulfinyläthyl bedeutet.

15. Verbindungen gemäss Anspruch 14, dadurch gekennzeichnet, dass $R^{11}$ 2-Propenyl bedeutet.

16. Verbindungen gemäss einem der Ansprüche 13 bis 15, dadurch gekennzeichnet, dass $R^6$ niederes Alkyl, niederes Phenylalkyl oder niederes Alkoxyalkyl und $R^7$ niederes Alkyl oder niederes Phenylalkyl oder $R^6$ und $R^7$ zusammen mit dem Sauerstoffatom und dem Chiralitätszentrum einen 5- oder 6-gliedrigen, gegebenenfalls ein weiteres, mit dem Chiralitätszentrum nicht direkt verbundenes Sauerstoffatom enthaltenden und gegebenenfalls durch niederes Alkyl, niederes Alkoxy, Oxo oder niederes Spirocycloalkyl substituierten O-Heterocyclus bedeuten.

17. Verbindungen gemäss Anspruch 16, dadurch gekennzeichnet, dass $R^6$ und $R^7$ zusammen mit dem Sauerstoffatom und dem Chiralitätszentrum die Gruppe

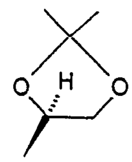

[(R)-2,2-Dimethyl-1,3-dioxolan-4-yl] bedeuten.

**Patentansprüche für den Vertragsstaat AT**

1. Verfahren zur Herstellung von optisch einheitlichen β-Lactamen der allgemeinen Formel

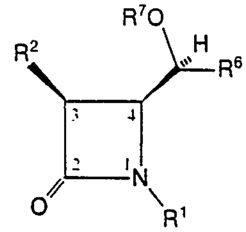

I

worin $R^1$ niederes 2- oder 1-Alkenyl oder eine Gruppe der Formel

$-CH_2-CH(OR^4)_2$ (a),

$-CH_2-CHO$ (b),

$-CO-CH(OH)_2$ (c) oder

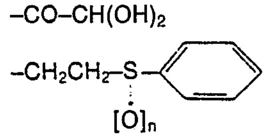

(d),

$R^4$ niederes Alkyl, n die Zahl 0 oder 1, $R^2$ Amino, Azido, Phthalimido, $ROCO-CH=C(CH_3)-NH-$ oder $Z-NH-$; R niederes Alkyl, Z eine leicht abspaltbare Acylgruppe und $R^6$ und $R^7$ je einen, gegebenenfalls miteinander verknüpften, gegebenenfalls Sauerstoff enthaltenden, über ein Kohlenstoffatom gebundenen niederen Kohlenwasserstoffrest bedeuten, wobei $R^1$ niederes 2- oder 1-Alkenyl oder eine der Gruppen (a), (c) und (d) bedeutet, wenn $R^2$ Amino bedeutet, und die mit nieder bezeichneten Reste bis zu 8 Kohlenstoffatome enthalten und den entsprechenden optischen Antipoden davon, dadurch gekennzeichnet, dass man ein reaktives Derivat einer Carbonsäure der allgemeinen Formel

$$R^{20}-CH^2-COOH \quad III$$

worin $R^{20}$ Azido, Phthalimido oder die Gruppe $ROCO-CH=C(CH_3)-NH-$ und R niederes Alkyl bedeuten, in Gegenwart einer Base mit einer Verbindung der allgemeinen Formel

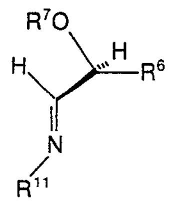

IV

worin $R^{11}$ niederes 2-Alkenyl oder eine der oben definierten Gruppen (a) und (d) bedeutet und $R^6$ und $R^7$ obige Bedeutung besitzen, oder dem entsprechenden optischen Antipoden davon zu einer Verbindung der allgemeinen Formel

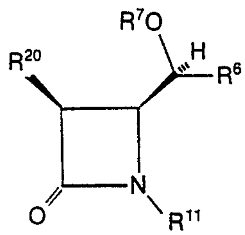

Ie

worin $R^{11}$, $R^{20}$, $R^6$ und $R^7$ obige Bedeutung besitzen, oder dem entsprechenden optischen Antipoden davon umsetzt und erwünschtenfalls eine als $R^{11}$ vorhandene niedere 2-Alkenylgruppe in die entsprechende 1-Alkenylgruppe oder eine als $R^{11}$ vorhandene Gruppe (a) in die Gruppe (b) oder (c) oder eine als $R^{11}$ vorhandene Gruppe (d) in die Vinylgruppe überführt, wobei man vorgängig oder anschliessend die Gruppe $R^{20}$ erwünschtenfalls in die Aminogruppe überführt und diese erwünschtenfalls mit einem eine leicht abspaltbare Acylgruppe abgebenden Mittel acyliert.

2. Verfahren gemäss Anspruch 1, dadurch gekennzeichnet, dass man Verbindungen gemäss Anspruch 1 herstellt, worin $R^1$ Vinyl, 2-Propenyl, 1-Propenyl, 2,2-Dimethoxyäthyl, 2,2-Diäthoxyäthyl, Formylmethyl, Dihydroxyacetyl, 2-Phenylthioäthyl oder 2-Phenylsulfinyläthyl bedeutet.

3. Verfahren gemäss Anspruch 2, dadurch gekennzeichnet, dass man Verbindungen gemäss Anspruch 2 herstellt, worin $R^1$ 2- oder 1-Propenyl bedeutet.

4. Verfahren gemäss einem der Ansprüche 1 bis 3, dadurch gekennzeichnet, dass man Verbindungen gemäss einem der Ansprüche 1 bis 3 herstellt, worin $R^2$ Amino, Phthalimido, Benzyloxycarbonylamino, t-Butoxycarbonylamino oder 2-Methoxycarbonyl-1-methyl-vinylamino bedeutet.

5. Verfahren gemäss einem der Ansprüche 1 bis 4, dadurch gekennzeichnet, dass man Verbindungen gemäss einem der Ansprüche 1 bis 4 herstellt, worin $R^6$ niederes Alkyl, niederes Phenylalkyl oder niederes Alkoxyalkyl und $R^7$ niederes Alkyl oder niederes Phenylalkyl oder $R^6$ und $R^7$ zusammen mit dem Sauerstoffatom und dem Chiralitätszentrum einen 5- oder 6-gliedrigen, gegebenenfalls ein weiteres, mit dem Chiralitätszentrum nicht direkt verbundenes Sauerstoffatom enthaltenden und gegebenenfalls durch niederes Alkyl, niederes Alkoxy, Oxo oder niederes Spirocycloalkyl substituierten O-Heterocyclus bedeuten.

6. Verfahren gemäss Anspruch 5, dadurch gekennzeichnet, dass man Verbindungen gemäss Anspruch 5 herstellt, worin $R^6$ und $R^7$ zusammen mit dem Sauerstoffatom und dem Chiralitätszentrum die Gruppe

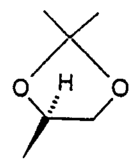

[(R)-2,2-Dimethyl-1,3-dioxolan-4-yl] bedeuten.

7. Verfahren gemäss Anspruch 1, dadurch gekennzeichnet, dass man N-[cis-(3S,4S)-1-Allyl-4-[(R)-2,2-dimethyl-1,3-dioxolan-4-yl)-2-oxo-3-Azetidinyl]phthalimid herstellt.

8. Verfahren gemäss Anspruch 1, dadurch gekennzeichnet, dass man N-[cis-(3S,4S)-4-[(R)-2,2-dimethyl-1,3-dioxolan-4-yl)-2-oxo-1-(1-propenyl)-3-azetidinyl]phthalimid herstellt.

9. Verfahren gemäss Anspruch 1, dadurch gekennzeichnet, dass man Benzyl-cis-(3S,4S)-4-[(R)-2,2-dimethyl-1,3-dioxolan-4-yl)-2-oxo-1-(1-propenyl)-3-azetidincarbamat herstellt.

10. Optisch einheitliche β-Lactame der allgemeinen Formel

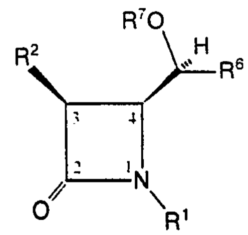

I

worin $R^1$ niederes 2- oder 1-Alkenyl oder eine Gruppe der Formel

$-CH_2-CH(OR^4)_2$ (a),

$-CH_2-CHO$ (b),

$-CO-CH(OH)_2$ (c) oder

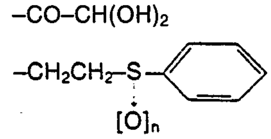

(d),

$R^4$ niederes Alkyl, n die Zahl 0 oder 1, $R^2$ Amino, Azido, Phthalimido, $ROCO-CH=C(CH_3)-NH-$ oder $Z-NH-$; R niederes Alkyl, Z eine leicht abspaltbare Acylgruppe und $R^6$ und $R^7$ je einen, gegebenenfalls miteinander verknüpften, gegebenenfalls Sauerstoff enthaltenden, über ein Kohlenstoffatom gebundenen niederen Kohlenwasserstoffrest bedeuten, wobei $R^1$ niederes 2- oder 1-Alkenyl oder eine der Gruppen (a), (c) und (d) bedeutet, wenn $R^2$ Amino bedeutet, und die mit nieder bezeichneten Reste bis zu 8 Kohlenstoffatome enthalten und die entsprechenden optischen Antipoden davon.

11. Verbindungen gemäss Anspruch 10, dadurch gekennzeichnet, dass $R^1$ Vinyl, 2-Propenyl, 1-Propenyl, 2,2-Dimethoxyäthyl, 2,2-Diäthoxyäthyl, Formylmethyl, Dihydroxyacetyl, 2-Phenylthioäthyl oder 2-Phenylsulfinyläthyl bedeutet.

12. Verbindungen gemäss Anspruch 11, dadurch gekennzeichnet, dass $R^1$ 2- oder 1-Propenyl bedeutet.

13. Verbindungen gemäss einem der Ansprüche 10 bis 12, dadurch gekennzeichnet, dass $R^2$ Amino, Phthalimido, Benzyloxycarbonylamino, t-Butoxycarbonylamino oder 2-Methoxycarbonyl-1-methyl-vinylamino bedeutet.

14. Verbindungen gemäss einem der Ansprüche 10 bis 13, dadurch gekennzeichnet, dass $R^6$ niederes Alkyl, niederes Phenylaklyl oder niederes Alkoxyalkyl und $R^7$ niederes Alkyl oder niederes Phenylalkyl oder $R^6$ und $R^7$ zusammen mit dem Sauerstoffatom und dem Chiralitätszentrum einen 5- oder 6-gliedrigen, gegebenenfalls ein weiteres, mit dem Chiralitätszentrum nicht direkt verbundenes Sauerstoffatom enthaltenden und gegebenenfalls durch niederes Alkyl, niederes Alkoxy, Oxo oder niederes Spirocycloalkyl substituierten O-Heterocyclus bedeuten.

15. Verbindungen gemäss Anspruch 14, dadurch gekennzeichnet, dass $R^6$ und $R^7$ zusammen mit dem Sauerstoffatom und dem Chiralitätszentrum die Gruppe

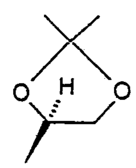

[(R)-2,2-Dimethyl-1,3-dioxolan-4-yl] bedeuten.

16. N-[cis-(3S,4S)-1-Allyl-4-[(R)-2,2-dimethyl-1,3-dioxolan-4-yl)-2-oxo-3-azetidinyl]phthalimid.

17. N-[cis-(3S,4S)-4-[(R)-2,2-dimethyl-1,3-dioxolan-4-yl)-2-oxo-1-(1-propenyl)- 3-azetidinyl]phthalimid.

18. Benzyl-cis-(3S,4S)-4-[(R)-2,2-dimethyl-1,3-dioxolan-4-yl)-2-oxo-1-(1-propenyl)-3-azetidincarbamat.

19. Verwendung von Verbindungen gemäss einem der Ansprüche 10 bis 18 zur Herstellung von antimikrobiell wirksamen, optisch einheitlichen β-Lactamen der allgemeinen Formel

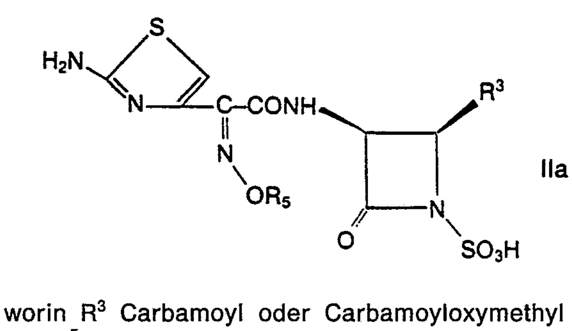

IIa

worin $R^3$ Carbamoyl oder Carbamoyloxymethyl und $R^5$ Wasserstoff, niederes Alkyl oder Carboxy-niederes Alkyl bedeuten, und den entsprechenden otpischen Antipoden davon.

20. Verwendung von Verbindungen gemäss einem der Ansprüche 10 bis 18 zur Herstellung von antimikrobiell wirksamen, optisch einheitlichen β-Lactamen gemäss Anspruch 18, worin $R^3$ Carbamoyloxymethyl und $R^5$ Carboxymethyl bedeuten.

21. Verbindungen der allgemeinen Formel

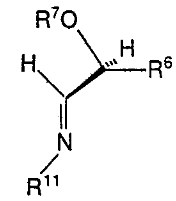

IV

worin $R^{11}$ niederes 2-Alkenyl oder eine der in Anspruch 10 definierten Gruppen (a) und (d) bedeutet und $R^6$ und $R^7$ die in Anspruch 10 angegebene Bedeutung besitzen, wobei $R^6$ und $R^7$ zusammen mit dem Sauerstoffatom nicht $-O-CH=CH-CH_2-CH_2-$ bedeuten, wenn $R^{11}$ Allyl bedeutet, und die entsprechenden optischen Antipoden davon.

22. Verbindungen gemäss Anspruch 21, dadurch gekennzeichnet, dass $R^{11}$ 2-Propenyl, 2,2-Dimethoxyäthyl, 2,2-Diäthoxyäthyl, 2-Phenylthioäthyl oder 2-Phenylsulfinyläthyl bedeutet.

23. Verbindungen gemäss Anspruch 22, dadurch gekennzeichnet, dass $R^{11}$ 2-Propenyl bedeutet.

24. Verbindungen gemäss einem der Ansprüche 21 bis 23, dadurch gekennzeichnet, dass $R^6$ niederes Alkyl, niederes Phenylalkyl oder niederes Alkoxyalkyl und $R^7$ niederes Alkyl oder niederes Phenylalkyl oder $R^6$ und $R^7$ zusammen mit dem Sauerstoffatom und dem Chiralitätszentrum einen 5- oder 6-gliedrigen, gegebenenfalls ein weiteres, mit dem Chiralitätszentrum nicht direkt verbundenes Sauerstoffatom enthaltenden und gegebenenfalls durch niederes Alkyl, niederes Alkoxy, Oxo oder niederes Spirocycloalkyl substituierten O-Heterocyclus bedeuten.

25. Verbindungen gemäss Anspruch 4, dadurch gekennzeichnet, dass $R^6$ und $R^7$ zusammen mit dem Sauerstoffatom und dem Chiralitätszentrum die Gruppe

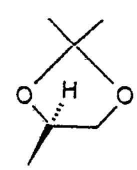

[(R)-2,2-Dimethyl-1,3-dioxolan-4-yl] bedeuten.

**Claims for the Contracting States: BE, CH, DE, FR, GB, IT, LI, LU, NL, SE.**

1. Optically uniform β-lactams of the general formula

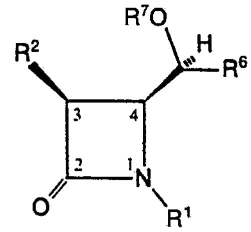

I

wherein $R^1$ signifies lower 2- or 1-alkenyl or a group of the formula

$-CH_2-CH(OR^4)_2$ (a),

$-CH_2-CHO$ (b),

$-CO-CH(OH)_2$ (c) or

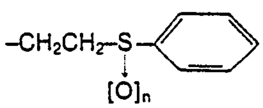

(d),

$R^4$ signifies lower alkyl, n signifies the number 0 or 1, $R^2$ signifies amino, azido, phthalimido. $ROCO-CH=C(CH_3)-NH-$ or Z–NH–; R signifies lower alkyl, Z signifies a readily cleavable acyl group and $R^6$ and $R^7$ each signify a lower hydrocarbon residue which optionally contains oxygen and which is attached via a carbon atom, said residues being optionally joined with one another, whereby $R^1$ signifies lower 2- or 1-alkenyl or one of the groups (a), (c) and (d) when $R^2$ signifies amino and the residues denoted as lower contain up ot 8 carbon atoms, and the corresponding optical antipodes thereof.

2. Compounds in accordance with claim 1, characterized in that $R^1$ signifies vinyl, 2-propenyl, 1-propenyl, 2,2-dimethoxyethyl, 2,2-diethoxyethyl, formylmethyl, dihydroxyacetyl, 2-phenylthioethyl or 2-phenylsulphinylethyl.

3. Compounds in accordance with claim 2, characterized in that $R^1$ signifies 2- or 1-propenyl.

4. Compounds in accordance with any one of claims 1 to 3, characterized in that $R^2$ signifies amino, phthalimido, benzyloxycarbonylamino, t-butoxycarbonylamino or 2-methoxycarbonyl-1-methyl-vinylamino.

5. Compounds in accordance with any one of claims 1 to 4, characterized in that $R^6$ signifies lower alkyl, lower phenylalkyl or lower alkoxyalkyl and $R^7$ signifies lower alkyl or lower phenylalkyl or $R^6$ and $R^7$ together with the oxygen atom and the centre of chirality signify a 5- or 6-membered O-heterocycle which optionally contains a further oxygen atom not directly linked with the centre of chirality and which is optionally substituted by lower alkyl, lower alkoxy, oxo or lower spirocycloalkyl.

6. Compounds in accordance with claim 5, characterized in that $R^6$ and $R^7$ together with the oxygen atom and the centre of chirality signify the group

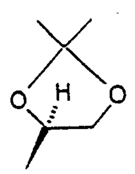

[(R)-2,2-dimethyl-1,3-dioxolan-4-yl].

7. N-[cis-(3S,4S)-1-Allyl-4-[(R)-2,2-dimethyl-1,3-dioxolan-4-yl)-2-oxo-3-azetidinyl]phthalimide.

8. N-[cis-(3S,4S)-4-[(R)-2,2-Dimethyl-1,3-dioxolan-4-yl)-2-oxo-1-(1-propenyl)-3-azetidinyl]phthalimide.

9. Benzyl cis-(3S,4S)-4-[(R)-2,2-dimethyl-1,3-dioxolan-4-yl)-2-oxo-1-(1-propenyl)-3-azetidinecarbamate.

10. A process for the manufacture of compounds in accordance with anyl one of claims 1 to 9, characterized by reacting a reactive derivative of a carboxylic acid of the general formula

$$R^{20}-CH_2-COOH \qquad III$$

wherein $R^{20}$ signifies azido, phthalimido or the group $ROCO-CH=C(CH_3)-NH-$ and R signifies lower alkyl, in the presence of a base with a compound of the general formula

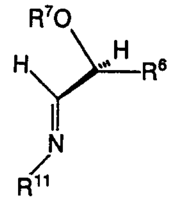

IV

wherein $R^{11}$ signifies lower 2-alkenyl or one of the groups (a) and (d) defined in claim 1 and $R^6$ and $R^7$ have the significance given in claim 1, or the corresponding optical antipode thereof to give a compound of the general formula

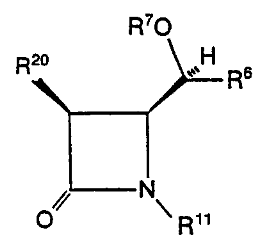

Ie

wherein $R^{11}$ and $R^{20}$ have the above significance and $R^6$ and $R^7$ have the significance given in claim 1, or the corresponding optical antipode thereof and, if desired, converting a lower 2-alkenyl group present as $R^{11}$ into the corresponding 1-alkenyl group or converting a group (a) present as $R^{11}$ into the group (b) or (c) or converting a group (d) present as $R^{11}$ into the vinyl group, whereby previously or subsequently the group $R^{20}$ is converted, if desired, into the amino group and, if desired, this is acylated with an agent yielding a readily cleavable axyl group.

11. The use of compounds in accordance with any one of claims 1 to 9 for the manufacture of antimicrobially active, optically uniform β-lactams of the general formula

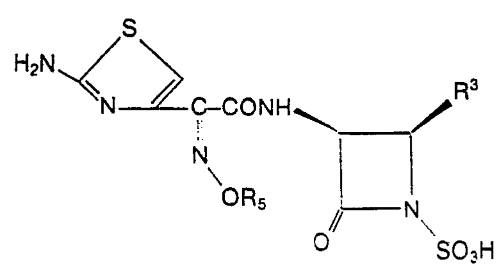

IIa

wherein $R^3$ signifies carbamoyl or carbamoyloxymethyl and $R^5$ signifies hydrogen, lower alkyl or carboxy-lower alkyl, and of the corresponding optical antipodes thereof.

12. The use of compounds in accordance with any one of claims 1 to 9 for the manufacture of

antimicrobially active, optically uniform β-lactams in accordance with claim 11, wherein $R^3$ signifies carbamoyloxymethyl and $R^5$ signifies carboxymethyl.

13. Compounds of the general formula

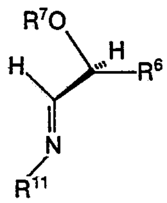

IV

wherein $R^{11}$ signifies lower 2-alkenyl or one of the groups (a) and (d) defined in claim 1 and $R^6$ and $R^7$ have the significance given in claim 1, whereby $R^6$ and $R^7$ together with the oxygen atom do not signify $-O-CH=CH-CH_2-CH_2-$ when $R^{11}$ signifies allyl, and the corresponding optical antipodes thereof.

14. Compounds in accordance with claim 13, characterized in that $R^{11}$ signifies 2-propenyl, 2,2-dimethoxyethyl, 2,2-diethoxyethyl, 2-phenylthioethyl or 2-phenylsulphinylethyl.

15. Compounds in accordance with claim 14, characterized in that $R^{11}$ signifies 2-propenyl.

16. Compounds in accordance with any one of claims 13 to 15, characterized in that $R^6$ signifies lower alkyl, lower phenylalkyl or lower alkoxyalkyl and $R^7$ signifies lower alkyl or lower phenylalkyl or $R^6$ and $R^7$ together with the oxygen atom and the centre of chirality signify a 5- or 6-membered O-heterocycle which optionally contains a further oxygen atom not directly linked with the centre of chirality and which is optionally substituted by lower alkyl, lower alkoxy, oxo or lower spriocycloalkyl.

17. Compounds in accordance with claim 16, characterized in that $R^6$ and $R^7$ together with the oxygen atom and the centre of chirality signify the group

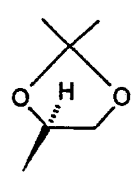

[(R)-2,2-dimethyl-1,3-dioxolan-4-yl].

**Claims for the Contracting State AT**

1. A process for the manufacture of optically uniform β-lactams of the general formula

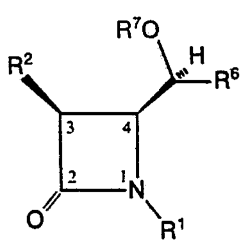

I

wherein $R^1$ signifies lower 2- or 1-alkenyl or a group of the formula

$-CH_2-CH(OR^4)_2$ (a),

$-CH_2-CHO$ (b),

$-CO-CH(OH)_2$ (c) or

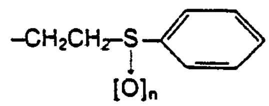

(d),

$R^4$ signifies lower alkyl, n signifies the number 0 or 1, $R^2$ signifies amino, azido, phthalimido, $ROCO-CH=C(CH_3)-NH-$ or Z-NH-; R signifies lower alkyl, Z signifies a readily cleavable acyl group and $R^6$ and $R^7$ each signify a lower hydrocarbon residue which optionally contains oxygen and which is attached via a carbon atom, said residues being optionally joined with one another, whereby $R^1$ signifies lower 2- or 1-alkenyl or one of the groups (a), (c) and (d) when $R^2$ signifies amino and the residues denoted as lower contain up to 8 carbon atoms, and of the corresponding optical antipodes thereof, characterized by reacting a reactive derivative of a carboxylic acid of the general formula

$$R^{20}-CH_2-COOH \quad III$$

wherein $R^{20}$ signifies azido, phthalimido or the group $ROCO-CH=C(CH_3)-NH-$ and R signifies lower alkyl, in the presence of a base with a compound or the general formula

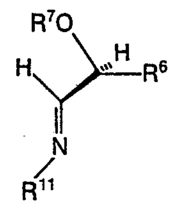

IV

wherein $R^{11}$ signifies lower 2-alkenyl or one of the groups (a) and (d) defined above and $R^6$ and $R^7$ have the above significance, or the corresponding optical antipode thereof to give a compound of the general formula

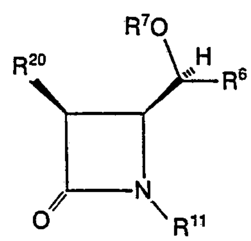

Ie

wherein $R^{11}$, $R^{20}$, $R^6$ and $R^7$ have the above significance, or the corresponding optical antipode thereof and, if desired, converting a lower 2-alkenyl group present as $R^{11}$ into the corresponding 1-alkenyl group or converting a group (a) present as $R^{11}$ into the group (b) or (c) or converting a group (d) present as $R^{11}$ into the vinyl group, whereby previously or subsequently the group $R^{20}$ is converted, if desired, into the amino group and, if desired, this is acylated with a readily cleavable acyl group.

2. A process in accordance with claim 1, characterized in that compounds in accordance with claim 1 in which $R^1$ signifies vinyl, 2-propenyl, 1-propenyl, 2,2-dimethoxyethyl, 2,2-diethoxyethyl, formylmethyl, dihydroxyacetyl, 2-phenylthioethyl or 2-phenylsulphinylethyl are manufactured.

3. A process in accordance with claim 2, characterized in that compounds in accordance with claim 2 in which $R^1$ signifies 2- or 1-propenyl are manufactured.

4. A process in accordance with any one of claims 1 to 3, characterized in that compounds in accordance with any one of claims 1 to 3 in which $R^2$ signifies amino, phthalimido, benzyloxycarbonylamino, t-butoxycarbonylamino or 2-methoxycarbonyl-1-methyl-vinylamino are manufactured.

5. A process in accordance with any one of claims 1 to 4, characterized in that compounds in accordance with any one of claims 1 to 4 in which $R^6$ signifies lower alkyl, lower phenylalkyl or lower alkoxyalkyl and $R^7$ signifies lower alkyl or lower phenylalkyl or $R^6$ and $R^7$ together with the oxygen atom and the centre of chirality signify a 5- or 6-membered O-heterocycle which optionally contains a further oxygen atom not directly linked with the centre of chirality and which is optionally substituted by lower alkyl, lower alkoxy, oxo or lower spirocycloalkyl are manufactured.

6. Compounds in accordance with claim 5, characterized in that compounds in accordance with claim 5 in which $R^6$ and $R^7$ together with the oxygen atom and the centre of chirality signify the group

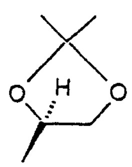

[(R)-2,2-dimethyl-1,3-dioxolan-4-yl] are manufactured.

7. A process in accordance with claim 1, characterized in that N-[cis-(3S,4S)-1-allyl-4-[(R)-2,2-dimethyl-1,3-dioxolan-4-yl)-2-oxo-3-azetidinyl]phthalimide is manufactured.

8. A process in accordance with claim 1, characterized in that N-[cis-(3S,4S)-4-[(R)-2,2-dimethyl-1,3-dioxolan-4-yl)-2-oxo-1-(1-propenyl)-3-azetidinyl]phthalimide is manufactured.

9. A process in accordance with claim 1, characterized in that benzyl cis-(3S,4S)-4-[(R)-2,2-dimethyl-1,3-dioxolan-4-yl)-2-oxo-1-(1-propenyl)-3-azetidinecarbamate is manufactured.

10. Optically uniform β-lactams of the general formula

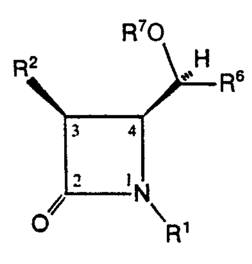

I

wherein $R^1$ signifies lower 2- or 1-alkenyl or a group of the formula

$-CH_2-CH(OR^4)_2$ (a),

$-CH_2-CHO$ (b),

$-CO-CH(OH)_2$ (c) or

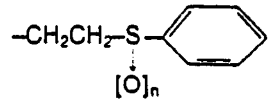

(d),

$R^4$ signifies lower alkyl, n signifies the number 0 or 1, $R^2$ signifies amino, azido, phthalimido, $ROCO-CH=C(CH_3)-NH-$ or $Z-NH-$; R signifies lower alkyl, Z signifies a readily cleavable acyl group and $R^6$ and $R^7$ each signify a lower hydrocarbon residue which optionally contains oxygen and which is attached via a carbon atom, said residues being optionally joined with one another, whereby $R^1$ signifies lower 2- or 1-alkenyl or one of the groups (a), (c) and (d) when $R^2$ signifies amino and the residues denoted as lower contain up to 8 carbon atoms, and the corresponding optical antipodes thereof.

11. Compounds in accordance with claim 10, characterized in that $R^1$ signifies vinyl, 2-propenyl, 1-propenyl, 2,2-dimethoxyethyl, 2,2-dietoxyethyl, formylmethyl, dihydroxyacetyl, 2-phenylthioethyl or 2-phenylsulphinylethyl.

12. Compounds in accordance with claim 11, characterized in that $R^1$ signifies 2- or 1-propenyl.

13. Compounds in accordance with any one of claims 10 to 12, characterized in that $R^2$ signifies amino, phthalimido, benzyloxycarbonylamino, t-butoxycarbonylamino or 2-methoxycarbonyl-1-methyl-vinylamino.

14. Compounds in accordance with any one of claims 10 to 13, characterized in that $R^6$ signifies lower alkyl, lower phenylalkyl or lower alkoxyalkyl and $R^7$ signifies lower alkyl or lower phenylalkyl or $R^6$ and $R^7$ together with the oxygen atom and the centre of chirality signify a 5- or 6-membered O-heterocycle which optionally contains a further oxygen atom not directly linked with the centre of chirality and which is optionally substituted by lower alkyl, lower alkoxy, oxo or lower spirocycloalkyl.

15. Compounds in accordance with claim 14, characterized in that $R^6$ and $R^7$ together with the oxygen atom and the centre of chirality signify the group

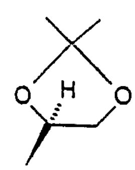

[(R)-2,2-dimethyl-1,3-dioxolan-4-yl].

16. N-[cis-(3S,4S)-1-Allyl-4-[(R)-2,2-dimethyl-1,3-dioxolan-4-yl)-2-oxo-3-azetidinyl]phthalimide.

17. N-[cis-(3S,4S)-4-[(R)-2,2-Dimethyl-1,3-dioxolan-4-yl)-2-oxo-1-(1-propenyl)-3-azetidinyl]phtalimide.

18. Benzyl cis-(3S,4S)-4-[(R)-2,2-dimethyl-1,3-dioxolan-4-yl)-2-oxo-1-(1-propenyl(-3-azetidinecarbamate.

19. The use of compounds in accordance with any one of claims 10 to 18 for the manufacture of antimicrobially active, optically uniform β-lactams of the general formula

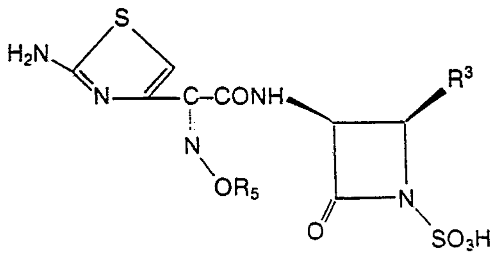

IIa

wherein $R^3$ signifies carbamoyl or carbamoyloxymethyl and $R^5$ signifies hydrogen, lower alkyl or carboxy-lower alkyl, and of the corresponding optical antipodes thereof.

20. The use of compounds in accordance with any one of claims 10 to 18 for the manufacture of antimicrobially active, optically uniform β-lactams in accordance with claim 18, wherein $R^3$ signifies carbamoyloxymethyl and $R^5$ signifies carboxymethyl.

21. Compounds of the general formula

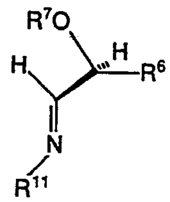

IV

wherein $R^{11}$ signifies lower 2-alkenyl or one of the groups (a) and (d) defined in claim 10 and $R^6$ and $R^7$ have the significance given in claim 10, whereby $R^6$ and $R^7$ together with the oxygen atom do not signify $-O-CH=CH-CH_2-CH_2-$ when $R^{11}$ signifies allyl, and the corresponding optical antipodes thereof.

22. Compounds in accordance with claim 21, characterized in that $R^{11}$ signifies 2-propenyl, 2,2-dimethoxyethyl, 2,2-diethoxyethyl, 2-phenylthioethyl or 2-phenylsulphinylethyl.

23. Compounds in accordance with claim 22, characterized in that $R^{11}$ signifies 2-propenyl.

24. Compounds in accordance with any one of claims 21 to 23, characterized in that $R^6$ signifies lower alkyl, lower phenylalkyl or lower alkoxyalkyl and $R^7$ signifies lower alkyl or lower phenylalkyl or $R^6$ and $R^7$ together with the oxygen atom and the centre of chirality signify a 5- or 6-membered O-heterocycle which optionally contains a further oxygen atom not directly linked with the centre of chirality and which is optionally substituted by lower alkyl, lower alkoxy, oxo or lower spirocycloalkyl.

25. Compounds in accordance with claim 24, characterized in that $R^6$ and $R^7$ together with the oxygen atom and the centre of chirality signify the group

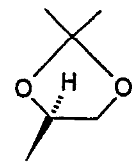

[(R)-2,2-dimethyl-1,3-dioxolan-4-yl].

**Revendications pour les Etats Contractants: BE, CH, DE, FR, GB, IT, LI, LU, NL, SE**

1. β-lactames optiquement uniformes de formule générale

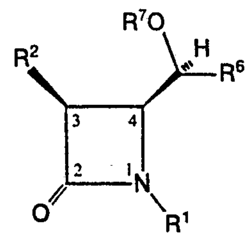

I

dans laquelle $R^1$ représente un 2- ou 1-alcényle inférieur ou un groupe de formule

$-CH_2-CH(OR^4)_2$ (a),

$-CH_2-CHO$ (b),

$-CO-CH(OH)_2$ (c) ou

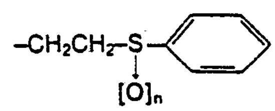

(d),

$R^4$ représente un alcoyle inférieur, n le nombre 0 ou 1, $R^2$ représente un amino, azido, phtalimido, $ROCO-CH=C(CH_3)-NH-$ ou Z–NH–; R représente un alcoyle inférieur, Z un groupe acyle facilement séparable et $R^6$ et $R^7$ chacun un radical hydrocarboné inférieur, éventuellement reliés entre eux, contenant éventuellement un oxygène, liés par l'intermédiaire d'un atome de carbone, où $R^1$ représente un groupe 2- ou 1-alcényle inférieur ou l'un des groupes (a), (c) et (d) lorsque $R^2$ représente un amino, et les radicaux décrits comme inférieurs contiennent jusqu'à 8 atomes de carbone et leurs antipodes optiques correspondants.

2. Composés selon la revendication 1, caractérisés en ce que $R^1$ représente un vinyle, 2-propényle, 1-propényle, 2,2-diméthoxyéthyle, 2,2-diéthoxyéthyle, formylméthyle, dihydroxyacétyle, 2-phénylthioéthyle ou 2-phénylsulfinyléthyle.

3. Composés selon la revendication 2, caractérisés en ce que $R^1$ représente un 2- ou 1-propényle.

4. Composés selon l'une des revendications 1 à 3, caractérisés en ce que $R^2$ représente un amino, phthalimido, benzyloxycarbonylamino, t-butoxycarbonylamino ou 2-méthoxycarbonyl-1-méthylvinylamino.

5. Composés selon l'une des revendications 1 à 4, caractérisés en ce que $R^6$ représente un alcoyle inférieur, phénylalcoyle inférieur ou alcoxyalcoyle

inférieur et $R^7$ représente un alcoyle inférieur ou un phénylalcoyle inférieur ou $R^6$ et $R^7$ ensemble avec l'atome d'oxygène et le centre de chiralité représentent un O-hétérocycle à 5 ou 6 chaînons, contenant éventuellement un autre atome d'oxygène non directement lié au centre de chiralité et éventuellement substitué par un alcoyle inférieur, alcoxy inférieur, oxo ou spirocycloalcoyle inférieur.

6. Composés selon la revendication 5, caractérisés en ce que $R^6$ et $R^7$ représentent ensemble avec l'atome d'oxygène et le centre de chiralité le groupe

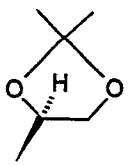

[(R)-2,2-diméthyl-1,3-dioxolan-4-yle].

7. N-[cis-(3S,4S)-1-allyl-4-[(R)-2,2-diméthyl-1,3-dioxolan-4-yl)-2-oxo-3-azétidinyl]phtalimide.

8. N-[cis-(3S,4S)-4-[(R)-,2-diméthyl-1,3-dioxolan-4-yl)-2-oxo-1-(1-propényl)-3-azétidinyl]phtalimide.

9. Benzyl-cis-(3S,4S)-4-[(R)-2,2-diméthyl-1,3-dioxolan-4-yl)-2-oxo-1-(1-propényl)-3-azétidinecarbamate.

10. Procédé de préparation de composés selon l'une des revendications 1 à 9, caractérisé en ce qu'on fait réagir un dérivé réactif d'un acide carboxylique de formule générale

$$R^{20}\text{–}CH_2\text{–}COOH \qquad III$$

où $R^{20}$ représente un azido, phtalimido ou le groupe ROCO–CH=C($CH_3$)–NH– et R représente un alcoyle inférieur, en présence d'une base avec un composé de formule générale

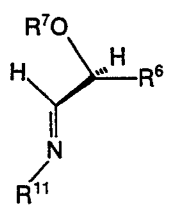

IV

où $R^{11}$ représente un 2-alcényle inférieur ou l'un des groupes (a) et (d) définis dans la revendication 1 et $R^6$ et $R^7$ ont la signification indiquée dans la revendication 1, ou son antipode optique correspondant pour donner un composé de formule générale

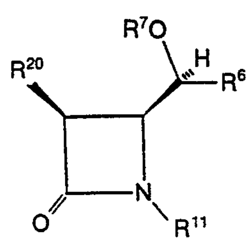

Ie

où $R^{11}$ et $R^{20}$ ont la signification donnée ci-dessus et $R^6$ et $R^7$ ont la signification donnée dans la revendication 1, ou son antipode optique correspondant et si on le désire on transforme un groupe 2-alcényle inférieur présent sous la forme de $R^{11}$ en le groupe 1-alcényle correspondant ou un groupe (a) présent sous la forme de $R^{11}$ en le groupe (b) ou (c) ou un groupe (d) présent sous la forme de $R^{11}$ en le groupe vinyle, opération dans laquelle si on le désire on transforme au préalable ou ultérieurement le groupe $R^{20}$ en le groupe amino et si on le désire on acyle ce dernier avec un agent donnant un groupe acyle facilement séparable.

11. Application de composés selon l'une des revendications 1 à 9 à la préparation de β-lactames optiquement uniformes, à action antimicrobienne, de formule générale

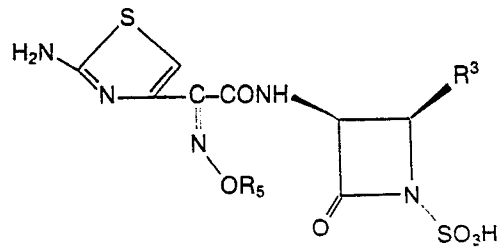

IIa

où $R^3$ représente un carbamoyle ou un carbamoyloxyméthyle et $R^5$ un hydrogène, un alcoyle inférieur ou un carboxy-alcoyle inférieur, et de leurs antipodes optiques correspondants.

12. Application de composés selon l'une des revendications 1 à 9 à la préparation de β-lactames optiquement uniformes à action antimicrobienne selon la revendication 11, où $R^3$ représente un carbamoyloxyméthyle et $R^5$ un carboxyméthyle.

13. Composés de formule générale

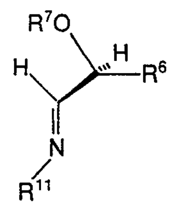

IV

où $R^{11}$ représente un 2-alcényle inférieur ou l'un des groupes (a) et (d) définis dans la revendication 1 et $R^6$ et $R^7$ ont la signification donnée dans la revendication 1, où $R^6$ et $R^7$ ensemble avec l'atome d'oxygène ne représentent pas $-O-CH=CH-CH_2-CH_2-$ lorsque $R^{11}$ représente un allyle, et leurs antipodes optiques correspondants.

14. Composés selon la revendication 13, caractérisés en ce que $R^{11}$ représente un 2-propényle, 2,2-diméthoxyéthyle, 2,2-diéthoxyéthyle, 2-phénylthioéthyle ou 2-phénylsulfinyléthyle.

15. Composés selon la revendication 14, caractérisés en ce que $R^{11}$ représente un 2-propényle.

16. Composés selon l'une des revendications 13 à 15, caractérisés en ce que $R^6$ représente un alcoyle inférieur, un phénylalcoyle inférieur ou un alcoxyalcoyle inférieur et $R^7$ représente un alcoyle inférieur ou un phénylalcoyle inférieur ou $R^6$

et $R^7$ ensemble avec l'atome d'oxygène et le centre de chiralité représentent un O-hétérocycle à 5 ou 6 chaînons contenant éventuellement un autre atome d'oxygène non directement lié au centre de chiralité et éventuellement substitué par un alcoyle inférieur, un alcoxy inférieur, un oxo ou un spirocycloalcoyle inférieur.

17. Composés selon la revendication 16, caractérisés en ce que $R^6$ et $R^7$ ensemble avec l'atome d'oxygène et le centre de chiralité représentent le groupe

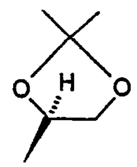

[(R)-2,2-diméthyl-1,3-dioxolan-4-yle].

**Revendications pour l'Etat Contractant: AT**

1. Procédé de préparation de β-lactames optiquement uniformes de formule générale

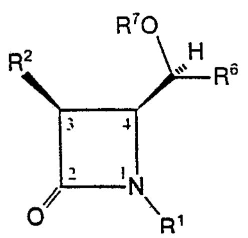

I

où $R^1$ représente un 2-ou 1-alcényle inférieur ou un groupe de formule

$-CH_2-CH(OR^4)_2$ (a),

$-CH_2-CHO$ (b),

$-CO-CH(OH)_2$ (c) ou

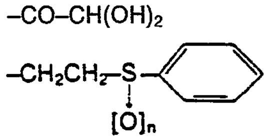

(d),

$R^4$ représente un alcoyle inférieur, n le nombre 0 ou 1, $R^2$ représente un amino, azido, phtalimido, $ROCO-CH=C(CH_3)-NH-$ ou Z–NH–; R représente un alcoyle inférieur, Z un groupe acyle facilement séparable et $R^6$ et $R^7$ représentent chacun un radical hydrocarboné inférieur, éventuellement reliés entre eux, contenant éventuellement de l'oxygène, liés par l'intermédiaire d'un atome de carbone, où $R^1$ représente un 2- ou 1-alcényle inférieur ou l'un des groupes (a), (c) et (d) lorsque $R^2$ représente un amino, et les radicaux décrits comme inférieurs contiennent jusqu'à 8 atomes de carbone, et de leurs antipodes optiques correspondants, caractérisé en ce qu'on fait réagir un dérivé réactif d'un acide carboxylique de formule générale

$R^{20}-CH_2-COOH$ III

où $R^{20}$ représente un azido, phtalimido ou le groupe $ROCO-CH=C(CH_3)-NH-$ et un alcoyle inférieur, en présence d'une base avec un composé de formule générale

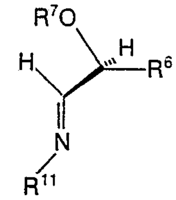

IV

où $R^{11}$ représente un 2-alcényle inférieur ou l'un des groupes (a) et (d) définis ci-dessus et $R^6$ et $R^7$ ont la définition donnée ci-dessus, ou l'antipode optique correspondant pour donner un composé de formule générale

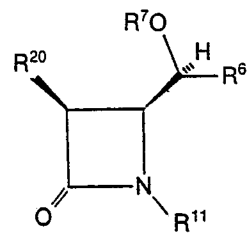

le

où $R^{11}$, $R^{20}$, $R^6$ et $R^7$ ont la signification donnée ci-dessus, où l'antipode optique correspondant et si on le désire on transforme un groupe 2-alcényle inférieur présent sous forme de $R^{11}$ en le groupe 1-alcényle correspondant ou un groupe (a) présent sous la forme de $R^{11}$ en le groupe (b) ou (c) ou un groupe (d) présent sous la forme de $R^{11}$ en le groupe vinyle, où si on le désire on transforme au préalable ou ensuite le groupe $R^{20}$ en le groupe amino et si on le désire on acyle ce dernier avec un agent donnant un groupe acyle facilement séparable.

2. Procédé selon la revendication 1, caractérisé en ce qu'on prépare des composés selon la revendication 1 où $R^1$ représente un vinyle, 2-propényle, 1-propényle, 2,2-diméthoxyéthyle, 2,2-diéthoxyéthyle, formylméthyle, dihydroxyacétyle, 2-phénylthioéthyle ou 2-phénylsulfinyléthyle.

3. Procédé selon la revendication 2, caractérisé en ce qu'on prépare des composés selon la revendication 2 où $R^1$ représente un 2- ou 1-propényle.

4. Procédé selon l'une des revendications 1 à 3, caractérisé en ce qu'on prépare des composés selon l'une des revendications 1 à 3 où $R^2$ représente un amino, phtalimido, benzyloxycarbonylamino, t-butoxycarbonylamino ou 2-méthylcarbonyl-1-méthylvinylamino.

5. Procédé selon l'une des revendications 1 à 4, caractérisé en ce qu'on prépare des composés selon l'une des revendications 1 à 4 où $R^6$ représente un alcoyle inférieur, phénylalcoyle inférieur ou alcoxyalcoyle inférieur, et $R^7$ représente un alocyle inférieur ou un phénylalcoyle inférieur ou $R^6$ et $R^7$ ensemble avec l'atome d'oxygène et le centre de chiralité représentent un O-hétérocycle à 5 ou 6 chaînons, contenant éventuellement un autre atome d'oxygène non directement lié au centre de chiralité et éventuellement substitué par

un alcoyle inférieur, alcoxy inférieur, oxo ou spirocycloalcoyle inférieur.

6. Procédé selon la revendication 5, caractérisé en ce qu'on prépare des composés selon la revendication 5, où $R^6$ et $R^7$ ensemble avec l'atome d'oxygène et le centre de chiralité représentent le groupe

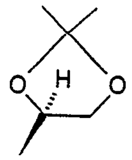

[(R)-2,2-diméthyl-1,3-dioxolan-4-yle].

7. Procédé selon la revendication 1, caractérisé en ce qu'on prépare le N-[cis-(3S,4S)-allyl-4-[(R)-2,2-diméthyl-1,3-dioxolan-4-yl)-2-oxo-3-azétidinyl]-phtalimide.

8. Procédé selon la revendication 1, caractérisé en ce qu'on prépare le N-[cis-(3S,4S)-4-[(R)-2,2-diméthyl-1,3-dioxolan-4-yl)-2-oxo-1-(1-propényl)-3-azétidinyl]phtalimide.

9. Procédé selon la revendication 1, caractérisé en ce qu'on prépare le benzyl-cis-(3S,4S)-4-[(R)-2,2,-diméthyl-1,3-dioxolan-4-yl)-2-oxo-1-(1-propényl)-3-azétidinecarbamate.

10. β-lactames optiquement uniformes de formule générale

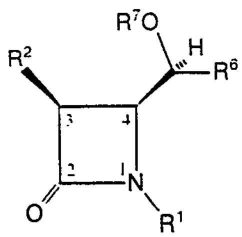

I

où $R^1$ représente un 2- ou 1-alcényle inférieur ou un groupe de formule

$-CH_2-CH(OR^4)_2$ (a),

$-CH_2-CHO$ (b),

$-CO-CH(OH)_2$ (c) ou

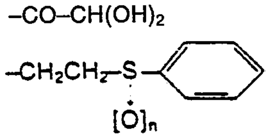

(d),

$R^4$ représente un alcoyle inférieur, n le nombre 0 ou 1, $R^2$ représente un amino, azido, phtalimido, $ROCO-CH=C(CH_3)-NH-$ ou Z-NH-; R représente un alcoyle inférieur, Z un groupe acyle facilement séparable et $R^6$ et $R^7$ chacun un radical hydrocarboné inférieur, éventuellement liés entre eux, contenant éventuellement un oxygène, liés par l'intermédiaire d'un atome de carbone, où $R^1$ représente un 2- ou 1-alcényle inférieur ou l'un des groupes (a), (c) et (d) lorsque $R^2$ représente un amino, et les radicaux décrits comme inférieurs contiennent jusqu'à 8 atomes de carbone et leurs antipodes optiques correspondants.

11. Composés selon la revendication 10, caractérisés en ce que $R^1$ représente un vinyle, 2-propényle, 1-propényle, 2,2-diméthoxyéthyle, 2,2-diéthoxyéthyle, formylméthyle, dihydroxyacétyle, 2-phénylthioéthyle ou 2-phénylsulfinyléthyle.

12. Composés selon la revendication 11, caractérisés en ce que $R^1$ représente un 2- ou 1-propényle.

13. Composés selon l'une des revendications 10 à 12, caractérisés en ce que $R^2$ représente un amino, phtalimido, benzyloxycarbonylamino, t-butoxycarbonylamino ou 2-méthoxycarbonyl-1-méthyl-vinylamino.

14. Composés selon l'une des revendications 10 à 13, caractérisés en ce que $R^6$ représente un alcoyle inférieur, phénylalcoyle inférieur ou alcoxyalcoyle inférieur et $R^7$ représente un alcoyle inférieur ou phénylalcoyle inférieur ou $R^6$ et $R^7$ avec l'atome d'oxygène et le centre de chiralité représentent un O-hétérocycle à 5 ou 6 chaînons, contenant éventuellement un autre atome d'oxygène non directement lié au centre de chiralité et éventuellement substitué par un alcoyle inférieur, alcoxy inférieur, oxo ou spirocycloalcoyle inférieur.

15. Composés selon la revendication 14, caractérisés en ce que $R^6$ et $R^7$ ensemble avec l'atome d'oxygène et le centre de chiralité représentent le groupe

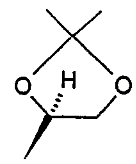

[(R)-2,2-diméthyl-1,3-dioxolan-4-yle].

16. N-[cis-(3S,4S)-1-allyl-4-[(R)-2,2-diméthyl-1,3-dioxolan-4-yl)-2-oxo-3-azétidinyl]phtalimide.

17. N-[cis-(3S,4S)-4-[(R)-2,2-diméthyl-1,3-dioxolan-4-yl)-2-oxo-1-(1-propényl)-3-azétidinyl]phtalimide.

18. Benzyl-cis-(3S,4S)-4-[(R)-2,2,-diméthyl-1,3-dioxolan-4-yl)-2-oxo-1-(1-propényl)-3-azétidine carbamate.

19. Application de composés selon l'une des revendications 10 à 18 à la préparation de β-lactames optiquement uniformes à activité antimicrobienne de formule générale

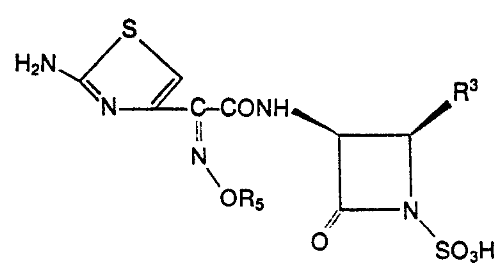

IIa

où $R^3$ représente un carbamoyle ou un carbamoyloxyméthyle et $R^5$ un hydrogène, un alcoyle inférieur ou un carboxy-alcoyle inférieur, et de leurs antipodes optiques correspondants.

20. Application de composés selon l'une des revendications 10 à 18 à la préparation de β-lactames optiquement uniformes à action antimicrobienne selon la revendication 18; où $R^3$ représente un carbamoyloxyméthyle et $R^5$ un carboxyméthyle.

21. Composés de formule générale

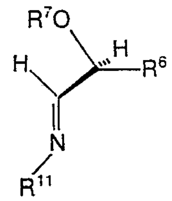

IV

où $R^{11}$ représente un 2-alcényle inférieur ou l'un des groupes (a) et (d) définis dans la revendication 10 et $R^6$ et $R^7$ ont la signification donnée dans la revendication 10, où $R^6$ et $R^7$ ensemble avec l'atome d'oxygène ne représentent pas $-O-CH=CH-CH_2-CH_2-$ lorsque $R^{11}$ représente un allyle, et leurs antipodes optiques correspondants.

22. Composés selon la revendication 21, caractérisés en ce que $R^{11}$ représente un 2-propényle, 2,2-diméthoxyéthyle, 2,2-diéthoxyéthyle, 2-phénylthioéthyle ou 2-phénylsulfinyléthyle.

23. Composés selon la revendication 22, caractérisés en ce que $R^{11}$ représente un 2-propényle.

24. Composés selon l'une des revendications 21 à 23, caractérisés en ce que $R^6$ représente un alcoyle inférieur, un phénylalcoyle inférieur ou un alcoxyalcoyle inférieur et $R^7$ un alcoyle inférieur ou un phénylalcoyle inférieur ou $R^6$ et $R^7$ représentent ensemble avec l'atome d'oxygène et le centre de chiralité un O-hétérocycle à 5 ou 6 chaînons, contenant éventuellement un autre atome d'oxygène non directement lié avec le centre de chiralité et éventuellement substitué par un alcoyle inférieur, alcoxy inférieur, oxo ou spirocycloalcoyle inférieur.

25. Composés selon la revendication 24, caractérisés en ce que $R^6$ et $R^7$ représentent avec l'atome d'oxygène et le centre de chiralité le groupe

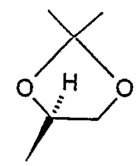

[(R)-2,2-diméthyl-1,3-dioxolan-4-yle].